# EUROPEAN PATENT SPECIFICATION

(11) **EP 1 730 526 B1**
(45) Date of publication and mention of the grant of the patent: **06.01.2010**
(21) Application number: 05725848.5
(22) Date of filing: 18.03.2005
(51) Int. Cl.: G01N 33/53, G01N 33/566, G01N 33/58, G01N 33/542

(54) **FLUORESCENCE POLARIZATION ASSAY**
FLUORESZENZPOLARISATIONSASSAY
ANALYSE PAR POLARISATION DE FLUORESCENCE

(30) Priority: 18.03.2004 US 554183 P
(43) Date of publication of application: 13.12.2006
(73) Proprietor: TransTech Pharma, Inc., High Point, NC 27265 (US)
(72) Inventor: MJALLI, Adnan, M.M., Oak Ridge, North Carolina 27310 (US); WEBSTER, Jeffrey, C., Jamestown, North Carolina 27282 (US)
(74) Representative: Bailey, Lindsey
(86) International application number: PCT/US2005/009005
(87) International publication number: WO 2005/089454

(56) References cited:
- EP-A- 1 415 997
- WO-A-01/92892
- WO-A-03/008446
- US-A1- 2003 087 302
- US-A1- 2003 087 302
- US-B1- 6 555 340
- OWICKI J C: "FLUORESCENCE POLARIZATION AND ANISOTROPY IN HIGH THROUGPUT SCREENING: PERSPECTIVES AND PRIMER" JOURNAL OF BIOMOLECULAR SCREENING, LARCHMONT, NY, US, vol. 5, no. 5, October 2000 (2000-10), pages 297-306, XP009007612 ISSN: 1087-0571
- BURKE T.J. ET AL.: 'Development and application of Fluorescence Polarization Assays in Drug Discovery' COMBINATORIAL CHEMISTRY & HIGH THROUGHPUT SCREENING vol. 6, 2003, pages 183 - 194, XP008050421

## Description

### FIELD OF THE INVENTION

The present invention relates to the use of methods and systems that use fluorescence polarization to identify compounds that have the ability to modulate the Receptor for Advanced Glycated Endproducts (RAGE).

### BACKGROUND

Incubation of proteins or lipids with aldose sugars results in nonenzymatic glycation and oxidation of amino groups on proteins to form Amadori adducts. Over time, the adducts undergo additional rearrangements, dehydrations, and cross-linking with other proteins to form complexes known as Advanced Glycosylation End Products (AGEs). Factors which promote formation of AGEs include delayed protein turnover (*e.g*. as in amyloidoses), accumulation of macromolecules having high lysine content, and high blood glucose levels (*e.g.* as in diabetes) (Hori et al., J. Biol. Chem. 270: 25752-761, (1995)). AGEs have been implicated in a variety of disorders including complications associated with diabetes and normal aging.

AGEs display specific and saturable binding to cell surface receptors on monocytes, macrophages, endothelial cells of the microvasculature, smooth muscle cells, mesengial cells, and neurons. The Receptor for Advanced Glycated Endproducts (RAGE) is a member of the immunoglobulin super family of cell surface molecules. The extracellular (N-terminal) domain of RAGE includes three immunoglobulin-type regions: one V (variable) type domain followed by two C-type (constant) domains (Neeper et al., J. Biol. Chem., 267:14998-15004 (1992); Schmidt et al., Circ. (Suppl.) 96#194 (1997)). A single transmembrane spanning domain and a short, highly charged cytosolic tail follow the extracellular domain. The N-terminal, extracellular domain can be isolated by proteolysis of RAGE or by molecular biological approaches to generate soluble RAGE (sRAGE) comprised of the V and C domains.

RAGE is expressed in most tissues, and in particular, is found in cortical neurons during embryogenesis (Hori et al., J. Biol. Chem., 270:25752-761 (1995)). Increased levels of RAGE are also found in aging tissues (Schleicher et al., J. Clin. Invest., 99 (3): 457-468 (1997)), and the diabetic retina, vasculature and kidney (Schmidt et al., Nature Med., 1:1002-1004 (1995)). Activation of RAGE in different tissues and organs leads to a number of pathophysiological consequences. RAGE has been implicated in a variety of conditions including: acute and chronic inflammation (Hofmann et al., Cell 97:889-901 (1999)), the development of diabetic late complications such as increased vascular permeability (Wautier et al., J. Clin. Invest., 97:238-243 (1995)), nephropathy (Teillet et al., J. Am. Soc. Nephrol., 11:1488-1497 (2000)), atherosclerosis (Vlassara *et. al., The Finnish Medical Society DUODECIM,* Ann. Med., 28:419-426 (1996)), and retinopathy (Hammes et al., Diabetologia, 42:603-607 (1999)). RAGE has also been implicated in Alzheimer's disease (Yan et al., Nature, 382:685-691 (1996)), erectile dysfunction, and in tumor invasion and metastasis (Taguchi et al., Nature, 405:354-357 (2000)).

In addition to AGEs, other compounds can bind to, and modulate RAGE. In normal development, RAGE interacts with amphoterin, a polypeptide that mediates neurite outgrowth in cultured embryonic neurons (Hori *et al.,* 1995). RAGE has also been shown to interact with carboxymethyl lysine (CML), calgranulin-like ligands and β-amyloid (Yan et al., Nature, 389:589-595, (1997); Yan et al., Nature, 382:685-691 (1996); Yan et al., Proc. Natl. Acad. Sci., 94:5296-5301 (1997)).

Binding of ligands such as AGEs, S100/calgranulin, β-amyloid, CML (N^{ε}-Carboxymethyl lysine), and amphoterin to RAGE has been shown to modify expression of a variety of genes. For example, in many cell types, interaction between RAGE and its ligands generates oxidative stress, which thereby results in activation of the free radical sensitive transcription factor NF-κB, and the activation of NF-κB regulated genes, such as the cytokines IL-1β, TNF-α, and the like. In addition, several other regulatory pathways, such as those involving p21ras, MAP kinases, ERK1, and ERK2, have been shown to be activated by binding of AGEs and other ligands to RAGE. In fact, transcription of RAGE itself is regulated at least in part by NF-κB. Thus, an ascending and often detrimental spiral is fueled by a positive feedback loop initiated by ligand binding.

Antagonizing the binding of physiological ligands to RAGE may be a logical target for down-regulation of the pathophysiological changes brought about by excessive concentrations of AGEs and other ligands for RAGE. By reducing binding of endogenous ligands to RAGE, symptoms associated with RAGE-mediated disorders may be reduced.

International patent application WO01/92892 discloses methods to detect modulators of RAGE and ligands but the methods are not based on fluorescent polarisation assays.

The review with the title "Fluorescence Polarisation and Anisotropy in High Throughput Screening: Perspectives and Primer, Owicki, J.C., Journal of Biomolecular Screening, Vol. 5, No. 5, 2000) discloses a variety of receptor ligand pairs that can be used in screening assays. However, RAGE and β-amyloid are not among the pairs disclosed.

### SUMMARY

The invention in its broadest form is defined in independent claims 1 and 23:
Claim 1:
   A method for detection of RAGE modulators comprising:
      (a) providing
         (i) a RAGE polypeptide comprising a ligand binding domain of RAGE,
         (ii) a fluorescent RAGE ligand, wherein the fluorescent RAGE ligand comprises an amyloid beta polypeptide, and
         (iii) a compound of interest;
      (b) adding the compound of interest and the fluorescent RAGE ligand to the RAGE polypeptide; and
      (c) measuring the polarization of the fluorescent RAGE ligand; and
      (d) correlating the level of polarization to the amount of the fluorescent RAGE ligand that is bound to the RAGE polypeptide.
Claim 23:
   A system for detection of compounds that modulate the binding of a ligand to RAGE using the methods of claims 1-22 comprising individually packaged containers of;
      (a) a fluorescent RAGE ligand, wherein the fluorescent ligand comprises an amyloid beta polypeptide;
      (b) a RAGE polypeptide comprising a ligand binding domain of RAGE,; and
      (c) at least one assay reagent for dilution of (a) and (b) in the presence of a compound of interest, such that binding of the fluorescent RAGE ligand to the polypeptide comprising the ligand binding domain of RAGE may be quantified.

The present specification further discloses methods and systems that use fluorescence polarization to identify compounds that have the ability to modulate the Receptor for Advanced Glycated Endproducts (RAGE). The present specification may comprise assay methods and/or systems for the discovery of compounds that can modulate the binding of physiological ligands to RAGE.

In one embodiment, the present specification comprises a method for detection of RAGE modulators comprising providing (i) a RAGE polypeptide comprising a ligand binding domain of RAGE, (ii) a fluorescent RAGE ligand, and (iii) a compound of interest. The method may further comprise adding the compound of interest and the fluorescent RAGE ligand to the RAGE polypeptide, and measuring the polarization of the fluorescent RAGE ligand.

In yet another embodiment, the specification comprises a system for detection of RAGE modulators. The system may comprise individually packaged containers of: (a) a fluorescent RAGE ligand; (b) a polypeptide comprising a ligand binding domain of RAGE; and (c) at least one assay reagent for dilution of (a) and (b) in the presence of a compound of interest, such that binding of the fluorescent RAGE ligand to the polypeptide comprising the ligand binding domain of RAGE may be quantified. In one embodiment, the system may further comprise an unlabeled RAGE ligand having substantial binding affinity for the RAGE ligand binding domain as a positive control.

There may be various advantages that may be associated with particular embodiments of the present specification. For example, the methods and systems of the present invention may measure binding of physiological ligands to RAGE and the modification of such binding. Also, the methods and systems of the present invention may allow for quantifying the ability of a compound of interest to bind to RAGE, or to modulate the binding of a physiological ligand to RAGE.

By eliminating many of the washing steps associated with traditional binding assays, the methods and systems of the present invention may provide for high-throughput, automated screening of multiple compounds. Thus, the methods and systems of the present invention may provide for rapid analysis of potential RAGE modulators.

Also, because the methods and systems comprise assay reagents that display high affinity, quantifiable binding to RAGE, the assay may provide a sensitive and highly specific assay system for detecting compounds having the potential to bind to, and/or modulate RAGE.

Also, the methods and systems of the present invention may provide a safe and reliable assay system that is not limited by many of the safety and disposal concerns typical of systems that use radiolabeled reagents. The assay employs non-radiolabeled reagents which do not present a threat to laboratory personnel or the environment.

The method may utilize a full-length RAGE protein, or a RAGE polypeptide comprising the ligand binding domain of RAGE, as a sensitive *in vitro* reagent to screen large numbers of potential RAGE modulators to identify those molecules that may function *in vivo* to modulate a biological activity mediated by RAGE. By varying the nature of the fluorescent ligand used, it may be possible to identify modulators that vary in structure and physiological effect.

### BRIEF DESCRIPTION OF THE FIGURES

Various features, aspects and advantages of the present invention will become more apparent with reference to the following figures.
**FIG. 1** shows a schematic representation of using fluorescent polarization for measuring RAGE-ligand interactions, wherein panel **(A)** shows binding of a fluorescent ligand (triangle) to STAGE resulting in a higher millipolarization (mP) value for the fluorescent ligand, and panel **(B)** shows reduction of binding of a fluorescent ligand (triangle) to sRAGE by an unlabeled RAGE ligand (rectangle) with a concomitant reduction in millipolarization (mP), in accordance with alternate embodiments of the present invention.
**FIG. 2** shows **(A)** SEQ ID NO: 1, the amino acid sequence for human RAGE as reported in GenBank/EMBL database, accession number XM004205; **(B)** SEQ ID NO: 2, an amino acid sequence of human sRAGE; **(C)** SEQ ID NO: 3, a amino acid sequence for the V-domain of human RAGE; **(D)** SEQ ID NO: 4, an N-terminal fragment of the V-domain of human RAGE; and **(E)** SEQ ID NO: 7, an alternative amino acid sequence of human sRAGE, in accordance with alternate embodiments of the present invention.
**FIG. 3** shows SEQ ID NO: 5, the amino acid sequence of human amyloid-beta (1-40); and SEQ ID NO: 6, the amino acid sequence of human amyloid beta (1-42).
**FIG. 4** shows that various RAGE ligands can compete with fluorescent amyloid beta (F1-Amyloid beta) for binding to sRAGE in accordance with alternate embodiments of the present invention.
**FIG. 5** shows the effect of DMSO on binding of fluorescent amyloid beta to sRAGE in accordance with an embodiment of the present invention.
**FIG. 6** shows a schematic representation of complexing a RAGE polypeptide comprising a RAGE ligand binding domain (e.g., sRAGE) with an anti-RAGE antibody (e.g., Ab) as a means to increase the change in millipolarization for free fluorescent ligand (triangle) as compared to receptor-bound fluorescent ligand.
**FIG. 7** shows a plot of small organic molecule antagonists inhibiting the interaction between RAGE and fluorescent amyloid beta (1-40) in accordance with alternate embodiments of the present invention.

### DETAILED DESCRIPTION

In the following description of the invention "RAGE ligand" shall mean "RAGE ligand that comprises an amyloid beta polypeptide".

The present invention relates to the use of a high-throughput assay for the discovery of compounds that can modulate RAGE activity. Embodiments of the method measure the ability of a compound of interest to modulate binding of a fluorescent RAGE ligand to RAGE protein, or to a fragment of RAGE comprising a ligand binding domain, under conditions in which the physiological and structural integrity of the RAGE ligand binding domain is substantially maintained. Those compounds that are able to modulate ligand binding to RAGE may then be assessed for physiological activity.

In one embodiment, the present invention comprises a method to detect compounds than can modulate binding of a RAGE ligand to the ligand binding domain of RAGE. The method may comprise the steps of: (a) providing (i) a RAGE polypeptide comprising a ligand binding domain of RAGE, (ii) a fluorescent RAGE ligand; and (iii) a compound of interest; (b) adding the compound of interest and the fluorescent RAGE ligand to the RAGE polypeptide; and (c) measuring the polarization of the fluorescent RAGE ligand.

The method may further comprise correlating the level of polarization of the fluorescent RAGE ligand to the amount of fluorescent RAGE ligand that is bound to the RAGE polypeptide. In one embodiment, the polarization of the fluorescent RAGE ligand may increase when the fluorescent RAGE ligand binds to the RAGE polypeptide comprising a RAGE ligand binding domain. Thus, the millipolarization value for a fluorescent ligand that is free in solution may be lower than the millipolarization value for the same fluorescent ligand that is bound to a second molecule (e.g., a RAGE polypeptide). If an unlabeled compound of interest is able to effectively compete for binding to the RAGE polypeptide, the fluorescent RAGE ligand may be displaced from the RAGE polypeptide, thereby reducing the measured levels of polarization. In the case where the compound of interest completely displaces the fluorescent RAGE ligand, polarization may be reduced to the levels seen for free (i.e., unbound) fluorescent RAGE ligand.

The assay may provide a quantitative measure of binding affinity. For example, the assay may comprise measuring the amount of fluorescent RAGE ligand bound to the RAGE polypeptide in the presence of varying amounts of the compound of interest. In one embodiment, the affinity of the compound of interest for the RAGE ligand binding domain is quantified based on the amount of the compound of interest required to reduce the binding of the fluorescent RAGE ligand to the RAGE polypeptide by a predetermined amount. Thus, the system may comprise the step of comparing the amount of fluorescent RAGE ligand bound to the RAGE polypeptide in the presence of varying amounts of the compound of interest. If the compound of interest competitively displaces the fluorescent RAGE ligand from the RAGE ligand binding domain, the level of polarization may be expected to decrease as the amount of the unlabeled compound of interest increases.

The compound of interest may be compared to other compounds that have varying affinity for RAGE or a polypeptide comprising the ligand binding domain of RAGE. Thus, in one embodiment, the ability of the compound of interest to reduce binding of the fluorescent RAGE ligand to the RAGE polypeptide is compared to the ability of a second compound to reduce binding of the fluorescent RAGE ligand to the RAGE polypeptide. In one embodiment, the second compound does not substantially bind to the RAGE ligand binding domain, such that the second compound functions as a negative control for the measurement of binding to the RAGE ligand binding domain.

For example, the method of the present invention may comprise comparing the ability of the compound of interest to compete with a fluorescent RAGE ligand for binding to the RAGE ligand binding domain with a second compound that does not bind to the RAGE ligand binding domain with physiological specificity. In this way, the effects of the compound of interest may be interpreted. Thus, where the compound of interest displaces the fluorescent ligand from the RAGE polypeptide, but requires concentrations similar to displacement by a negative control compound, it may be that the compound of interest does not bind to the RAGE ligand binding domain with high affinity. In alternate embodiments, a negative control may be a molecule that exhibits a dissociation constant (Kd) for binding to RAGE of greater than 1 mM, or greater than 100 µM, or greater than 50 µM, or greater than 20 µM, or greater than 10 µM, or greater than 5 µM.

Alternatively, the second compound may have a substantial binding affinity for the RAGE ligand binding domain, such that the second compound functions as a positive control for the measurement of binding to the RAGE ligand binding domain. In this way, the relative affinity of the compound of interest as compared to the positive control compound for binding to RAGE may be assessed. Thus, where the compound of interest displaces the fluorescent ligand from the RAGE polypeptide at concentrations similar to displacement by a positive control compound, it may be that the compound of interest binds to the RAGE ligand binding domain with high affinity. In one embodiment, the positive control may comprise a small molecule RAGE antagonist that binds to RAGE with substantial binding affinity. In alternate embodiments, a positive control may be a molecule that exhibits a dissociation constant (Kd) for binding to RAGE of less than 1 µM, or less than 200 nM, or less than 50 nM, or less than 10 nM.

In one embodiment, the second compound may comprise an experimental molecule that is a chemical and/or structural variant of the compound of interest. By comparing the relative affinity of the compound of interest to a second compound that is a chemical and/or structural variant of the compound of interest, the effect of the particular chemical and/or structural variation on the binding affinity to the RAGE polypeptide may be determined. In this way, the assay may be used to define structure-activity relationships (SAR) for RAGE ligands.

The present invention may also comprise a system for the detection of compounds that have the ability to modulate ligand binding to RAGE and/or RAGE activity. In one embodiment, the present invention may comprise a system for detection of compounds that modulate the binding of a ligand to RAGE comprising individually packaged containers of: (a) a fluorescent RAGE ligand; (b) a RAGE polypeptide comprising a ligand binding domain of RAGE; and (c) at least one assay reagent for dilution of (a) and (b) in the presence of a compound of interest, such that binding of the fluorescent RAGE ligand to the polypeptide comprising the ligand binding domain of RAGE may be quantified.

The system may allow for the quantitative analysis of ligand binding to RAGE. Thus, in one embodiment, the system comprises a device to measure the polarization of the fluorescent ligand.

Using the systems of the present invention, the compound of interest may be compared to other compounds that have varying affinity for RAGE or a polypeptide comprising the ligand binding domain of RAGE. Thus, in one embodiment, the ability of the compound of interest to reduce binding of the fluorescent ligand to the RAGE polypeptide is compared to the ability of a compound comprising a predetermined binding affinity for the RAGE ligand binding domain to reduce binding of the fluorescent ligand to the RAGE polypeptide. In one embodiment, the compound comprising a predetermined binding affinity for the RAGE ligand binding domain does not substantially bind to the RAGE binding domain, such that the second compound functions as a negative control for the measurement of binding to the RAGE ligand binding domain. Alternatively, the second compound may comprise a predetermined binding affinity for the RAGE binding domain, such that the second compound functions as a positive control for the measurement of binding to the RAGE ligand binding domain. In one embodiment, the positive control may comprise a small molecule RAGE antagonist that binds to RAGE with substantial binding affinity.

The second compound may comprise an experimental molecule that is a chemical and/or structural variant of the compound of interest. By comparing the relative affinity of the compound of interest to a second compound that is a chemical and/or structural variant of the compound of interest, the effect of the particular chemical and/or structural variation on the binding affinity to the RAGE polypeptide may be determined. In this way, the assay may be used to define structure-activity relationships (SAR) for RAGE ligands. In one embodiment, the system may comprise a computer system and/or software to analyze the SAR results for various compounds of interest.

In one embodiment, the RAGE polypeptide comprising a RAGE ligand binding domain comprises a polypeptide having the amino acid sequence SEQ ID NO: 1, or a sequence 90% identical to SEQ ID NO: 1, or a fragment of SEQ ID NO: 1. In one embodiment, the sequence at least 90% identical to SEQ ID NO: 1 may comprise methionine (M) as the first amino acid rather than glycine (G).

The fragment of RAGE may comprise a polypeptide that is known to bind RAGE ligands with high affinity. The fragment of SEQ ID NO: 1 may comprise human sRAGE as defined by the amino acid sequence SEQ ID NO: 2, or a sequence 90% identical to SEQ ID NO: 2, or a fragment of SEQ ID NO: 2. In one embodiment, the sequence at least 90% identical to SEQ ID NO: 2 may comprise a recombinant sRAGE having methionine (M) as the first amino acid rather than glycine (G) (e.g., SEQ ID NO: 7).

Or, the fragment of SEQ ID NO: 1 may comprise the V domain of human RAGE as defined by the amino acid sequence SEQ ID NO: 3, or a sequence 90% identical to SEQ ID NO: 3, or a fragment of SEQ ID NO: 3. The V-domain has been identified as the ligand binding region ofsRAGE and RAGE (*see e.g.,* WO 99/18987).

Or a fragment of the V-domain may be used. In one example embodiment, the fragment of SEQ ID NO: 1 may comprise the V domain of human RAGE as defined by the amino acid sequence SEQ ID NO: 4, or a sequence 90% identical to SEQ ID NO: 4. Or a fragment of SEQ ID NO: 4 may be used as the RAGE ligand binding domain.

Both the methods and the systems of the present invention comprise an amyloid beta polypeptide. For example, in alternate embodiments, the fluorescent RAGE ligand may comprise human amyloid beta (1-40) having the amino acid sequence of SEQ ID NO: 5, or human amyloid beta (1-42) having the amino acid sequence of SEQ ID NO: 6. In one embodiment, the fluorophore may be attached to the amino terminus of the amyloid beta polypeptide to generate the fluorescent ligand. Or, the fluorophore may be attached to the carboxyl terminus of the amyloid beta polypeptide to generate the fluorescent ligand. In alternative embodiments, the fluorophore may be attached to other residues along the length of the polypeptide chain.

In another disclosed embodiment, the fluorescent RAGE ligand may comprise an advanced glycated endproduct. For example, the fluorescent RAGE ligand may comprise fluorescent carboxymethyllysine or a fluorescent carboxymethyllysine-modified AGE. Or, the fluorescent RAGE ligand may comprise calgranulin labeled with a fluorescent group or a fluorescent S-100b. Alternatively, the fluorescent RAGE ligand may comprise amphoterin or a small organic molecule comprising a molecular weight of less than 1000 Daltons.

A variety of fluorophores may be used to label the RAGE ligand of interest so long as the fluorophore does not substantially interfere with binding of the ligand to the RAGE ligand binding domain. In an embodiment, fluorecein is used to label the RAGE ligand. Alternatively, other fluorophores, such as, but not limited to, Lucifer yellow, eosin, propidium iodide, rhodamine (i.e., tetramethyl rhodamine or lissamine rhodamine B), cyanin 3 (Cy3), cyanin 5 (Cy5), Texas Red, or allophycocyanin may be used. The preferred excitation/emission maxima for the fluorophore may depend on the individual fluorophore as well as the nature of the coupling of the fluorophore to the RAGE ligand.

The fluorophore may be used to label the RAGE ligand using methods known in the art. For example, kits for labeling nucleic acids and peptides are commercially available (e.g., Molecular Probes, Inc., Eugene, OR; emp Biotech, GmbH). The fluorophore may be incorporated by chemical coupling of the fluorophore with the RAGE ligand. For example, in one embodiment, to make amyloid beta, the polypeptide is labeled at its N-terminal end with 5-carboxy-fluorescein. Fluorescent amyloid beta peptides are commercially available (e.g., rPeptide, Athens, GA; Biosource, Camarillo, CA).

In selected embodiments of either the method or the system of the present invention, the polypeptide comprising the binding domain of RAGE may be linked to, or allowed to complex with, a second, non-RAGE polypeptide. The second polypeptide may comprise an immunoglobulin domain. In one embodiment, the polypeptide comprising the ligand binding domain of RAGE may be allowed to complex with an antibody that recognizes and binds to, the polypeptide comprising the RAGE ligand binding domain. The antibody may comprise a polyclonal antibody. Or, the antibody may comprise a monoclonal antibody. By allowing the polypeptide comprising the ligand binding domain of RAGE to complex with an anti-RAGE antibody, the size of the construct comprising a RAGE binding site may be increased, thereby resulting in a larger increase in polarization for the fluorescent ligand upon binding to the RAGE polypeptide.

In one embodiment, an antibody to sRAGE may be used. For example, sRAGE may be used as the polypeptide comprising a RAGE ligand binding domain and anti-sRAGE antibody may be allowed to complex with the sRAGE.

The polypeptide comprising the ligand binding domain of RAGE may be allowed to complex with an anti-RAGE antibody prior to binding of the fluorescent ligand and/or the compound of interest. Or, the polypeptide comprising the ligand binding domain of RAGE may be allowed to complex with an anti-RAGE antibody substantially simultaneously with binding of the fluorescent RAGE ligand and/or the compound of interest. In yet another embodiment, the polypeptide comprising the ligand binding domain of RAGE may be allowed to complex with an anti-RAGE antibody after the RAGE polypeptide is allowed to with bind the fluorescent RAGE ligand and/or the compound of interest.

The invention relates to the use of a physiologically relevant binding assay to discover compounds that have the ability to modulate RAGE activity. In another embodiment, the present specification comprises compounds identified by the methods and/or systems of the invention as having the ability to modulate binding of the fluorescent ligand to RAGE. In one embodiment, the compound identified by the methods and/or the systems of the invention as having the ability to modulate binding of the fluorescent ligand to RAGE may bind to the ligand binding domain of RAGE. Or, the compound identified by the methods and/or the systems of the invention as having the ability to modulate binding of the fluorescent ligand to RAGE may interact with RAGE without binding to the ligand binding domain. Also, the compound identified by the methods and/or the systems of the invention as having the ability to modulate binding of the fluorescent ligand to RAGE may act as a RAGE agonist. Alternatively, the compound identified by the methods and/or the systems of the invention as having the ability to modulate binding of the fluorescent ligand to RAGE may act as a RAGE antagonist.

In an embodiment, the compound of interest may comprise a small organic molecule. For example, the compound may comprise a small organic molecule RAGE antagonist. In one embodiment, the small organic molecule may comprise a molecular weight of less than 1000 Daltons. In alternate embodiments, the small organic molecule may comprise a molecular weight that is in the range of about 400 to 900 Daltons, or from about 500 to about 800 Daltons.

Other types of potential RAGE modulators may be identified using the methods and/or systems of the present invention. Thus, the compound of interest and potential RAGE modulator may comprise a peptide. Alternatively, the compound of interest and potential RAGE modulator may comprise a peptidomimetic. Or, the compound of interest and potential RAGE modulator may comprise an inorganic compound. Also, the compound of interest and potential RAGE modulator may comprise a lipid. In another embodiment, the compound of interest and potential RAGE modulator may comprise a carbohydrate. Also, the compound of interest and potential RAGE modulator may comprise a nucleic acid.

The compounds identified using the methods and/or systems of the present invention may be formulated as compositions to be administered to subjects at risk of, or suffering from, a RAGE-mediated disease or syndrome. As described herein, RAGE has been shown to be involved in a multitude of cellular processes that may mediate a variety of disease states. By interfering with RAGE, and the processing of AGEs, compounds identified using the methods and/or systems of the present invention may be used as therapeutics.

For example, the compound identified using the methods and/or systems of the present invention may be used to treat amyloidoses. In one embodiment, the compound identified using the methods and/or systems of the present invention may be used to treat Alzheimer's disease. Or, the compound identified by the methods and/or systems of the present invention may be used to treat diabetes and/or a symptom of diabetic late complications. In another embodiment, the compound identified by the methods and/or systems of the present invention may be used to treat cancer. Alternatively or additionally, the compound identified by the methods and/or systems of the present invention may be used to treat inflammation. In another embodiment, the compound identified by the methods and/or systems of the present invention may be used to treat kidney failure. Or, the compound identified by the methods and/or systems of the present invention may be used to treat systemic lupus nephritis or inflammatory lupus nephritis. Also, the compound identified by the methods and/or systems of the present invention may be used to treat erectile dysfunction. In yet another embodiment, the compound identified by the methods and/or systems of the present invention may be used to treat stroke or heart attack.

In some embodiments the compositions used for treatment of RAGE-mediated disorders may comprise at least one additional therapeutic agent. For example, additional therapeutic agents comprising at least one of an alkylating agent, an antimetabolite, a plant alkaloid, an antibiotic, a hormone, a biologic response modifier, an analgesic, an NSAID, a DMARD, a glucocorticoid, a sulfonylurea, a biguanide, insulin, a cholinesterase inhibitor, an antipsychotic, an antidepressants, or an anticonvulsant may be used.

### Definitions

The following definitions may be used to understand the description herein. Unless defined otherwise, all technical and scientific terms used herein have the same meaning as commonly understood by one of ordinary skin in the art.

The term "a" or "an" as used herein may refer to more than one object unless the context clearly indicates otherwise. The term "or" is used interchangeably with the term "and/or" unless the context clearly indicates otherwise.

A "ligand" is a molecule that binds to a receptor to form a complex.

A "modulator" is a molecule that can physically interact with a second molecule and/or complex of molecules to cause a change in at least one characteristic of the second molecule and/or complex. The change may comprise a chemical change (e.g., formation of a chemical bond; alteration in net charge), a physical change (e.g., a change in the three-dimensional structure), and/or a change in the biological activity (e.g., an alteration of catalytic activity) of the second molecule and/or complex.

An "agonist" comprises a compound that binds to a receptor to form a complex that elicits a pharmacological response specific to the receptor involved.

An "antagonist" comprises a compound that binds to an agonist or a receptor to form a complex that does not give rise to a substantial pharmacological response and can inhibit the biological response induced by an agonist.

RAGE agonists may therefore bind to RAGE and stimulate RAGE-mediated cellular processes, and RAGE antagonists may inhibit RAGE-mediated processes from being stimulated by a RAGE agonist. For example, the cellular process stimulated by RAGE agonists may comprise activation of TNF-α gene transcription, or NF-κB gene transcription, or another cellular process.

"Polypeptide" and "protein" are used interchangeably herein to describe protein molecules that may comprise either partial or full-length proteins.

As used herein, "small organic molecules" are molecules of molecular weight less than 2,000 Daltons that contain at least one carbon atom.

A "polypeptide domain" comprises a region along a polypeptide that comprises an independent unit. Domains may be defined in terms of structure, sequence and/or biological activity. In one embodiment, a polypeptide domain may comprise a region of a protein that folds in a manner that is substantially independent from the rest of the protein. Domains may be identified using domain databases such as, but not limited to PFAM, PRODOM, PROSITE, BLOCKS, PRINTS, SBASE, ISREC PROFILES, SAMRT, and PROCLASS. A "ligand binding domain" is a region of a polypeptide that binds a ligand.

An, "immunoglobulin domain" is a sequence of amino acids that is structurally homologous, or identical, to a domain of an immunoglobulin. The length of the sequence of amino acids of an immunoglobulin domain may be any length. In one embodiment, an immunoglobulin domain may be less than 250 amino acids. In an example embodiment, an immunoglobulin domain may be about 80-150 amino acids in length.

The term "polyclonal antibodies" referst to antibodies that are a heterogeneous population of antibody molecules derived from the sera of animals immunized with the antigen of interest. Adjuvants such as Freund's (complete and incomplete), peptides, oil emulsions, lysolecithin, polyols, polyanions and the like may be used to increase the immune response. For example, a polyclonal antibody to sRAGE may prepared by injection of sRAGE supplemented with an adjuvant into rabbits using methods known in the art (*e.g.* Schmidt et al., J. Biol. Chem., 267:14987-14997 (1992)).

"Monoclonal antibodies" are homogeneous populations of antibodies to a particular antigen, and are generally obtained by any technique which provides for production of antibody by continuous cell lines in culture (*see e.g.* U.S. Patent No. 4,873,313). For example, sRAGE protein may used for production of monoclonal antibodies using methods known in the art (Zymed Laboratories, San Francisco, CA).

A "nucleic acid" is a polynucleotide such as deoxyribonucleic acid (DNA) or ribonucleic acid (RNA). The term is used to include single-stranded nucleic acids, doublestranded nucleic acids, and RNA and DNA made from nucleotide or nucleoside analogues.

The term "vector" refers to a nucleic acid molecule that may be used to transport a second nucleic acid molecule into a cell. In one embodiment, the vector allows for replication of DNA sequences inserted into the vector. The vector may comprise a promoter to enhance expression of the nucleic acid molecule in at least some host cells. Vectors may replicate autonomously (extrachromasomal) or may be integrated into a host cell chromosome. In one embodiment, the vector may comprise an expression vector capable of producing a protein derived from at least part of a nucleic acid sequence inserted into the vector.

The term "percent identical" refers to sequence identity between two amino acid sequences or between two nucleic acid sequences. Percent identity can be determined by aligning two sequences and refers to the number of identical residues (*i.e*., amino acid or nucleotide) at positions shared by the compared sequences. Sequence alignment and comparison may be conducted using the algorithms standard in the art (*e.g*. Smith and Waterman, Adv. Appl. Math. 2:482 (1981); Needleman and Wunsch, J. Mol. Biol. 48:443 (1970); Pearson and Lipman, Proc. Natl. Acad. Sci. (USA), 85:2444 (1988)) or by computerized versions of these algorithms (Wisconsin Genetics Software Package Release 7.0, Genetics Computer Group, 575 Science Drive, Madison, WI) publicly available as BLAST and FASTA. Also, ENTREZ, available through the National Institutes of Health, Bethesda MD, may be used for sequence comparison. In one embodiment, percent identity of two sequences may be determined using GCG with a gap weight of 1, such that each amino acid gap is weighted as if it were a single amino acid or nucleotide mismatch between the two sequences.

As is known in the art, conditions for hybridizing nucleic acid sequences to each other can be described as ranging from low to high stringency. Generally, highly stringent hybridization conditions refer to washing hybrids in low salt buffer at high temperatures. Hybridization may be to filter bound DNA using hybridization solutions standard in the art such as 0.5M NaHPO₄, 7% sodium dodecyl sulfate (SDS), at 65°C, and washing in 0.25 M NaHPO₄, 3.5% SDS followed by washing 0.1 x SSC/0.1% SDS at a temperature ranging from room temperature to 68°C depending on the length of the probe (*see e.g.* Ausubel, F.M. et al., Short Protocols in Molecular Biology, 4th Ed., Chapter 2, Jolm Wiley & Sons, N.Y). For example, a high stringency wash comprises washing in 6x SSC/0.05% sodium pyrophosphate at 37°C for a 14 base oligonucleotide probe, or at 48°C for a 17 base oligonucleotide probe, or at 55°C for a 20 base oligonucleotide probe, or at 60°C for a 25 base oligonucleotide probe, or at 65°C for a nucleotide probe about 250 nucleotides in length. Nucleic acid probes may be labeled with radionucleotides by end-labeling with, for example, [γ-³²P]ATP, or incorporation of radiolabeled nucleotides such as [α-³²P]dCTP by random primer labeling. Alternatively, probes may be labeled by incorporation of biotinylated or fluorescein labeled nucleotides, and the probe detected using Streptavidin or anti-fluorescein antibodies.

As used herein, the term "EC50" is defined as the concentration of an agent that results in 50% of a desired effect. For example, the EC50 of a therapeutic agent having a measurable biological effect may comprise the value at which the agent displays 50% of the biological effect.

As used herein, the term "IC50" is defined as the concentration of an agent that results in 50% inhibition of a measured effect. For example, the IC50 of an antagonist of RAGE binding may comprise the value at which the antagonist reduces ligand binding to the ligand binding domain of RAGE by 50%.

As used herein, a "chemical variant" of a compound of interest comprises a molecule that has at least one substitution of an atomic group in the compound of interest. As used herein, a "structural variant" of a compound of interest comprises a molecule that has the same empirical formula as the compound of interest, but a different three-dimensional configuration.

### Use of Fluorescence Polarization to Identify Compounds that Modulate RAGE Ligand Interactions

Thus, the present invention comprises methods and systems utilizing a polypeptide comprising the ligand binding domain of RAGE for the detection of compounds that have the ability to modulate binding of a physiological ligand to RAGE. The polypeptide comprising the ligand binding domain of RAGE may comprise RAGE protein, or a fragment of RAGE. In one embodiment, the method may comprise measuring displacement of a fluorescent RAGE ligand from a polypeptide comprising the ligand binding domain of RAGE by a compound of interest. If the compound of interest has the ability to displace the fluorescent ligands, the compound of interest may comprise a potential modulator of RAGE, and/or RAGE-mediated biological activity.

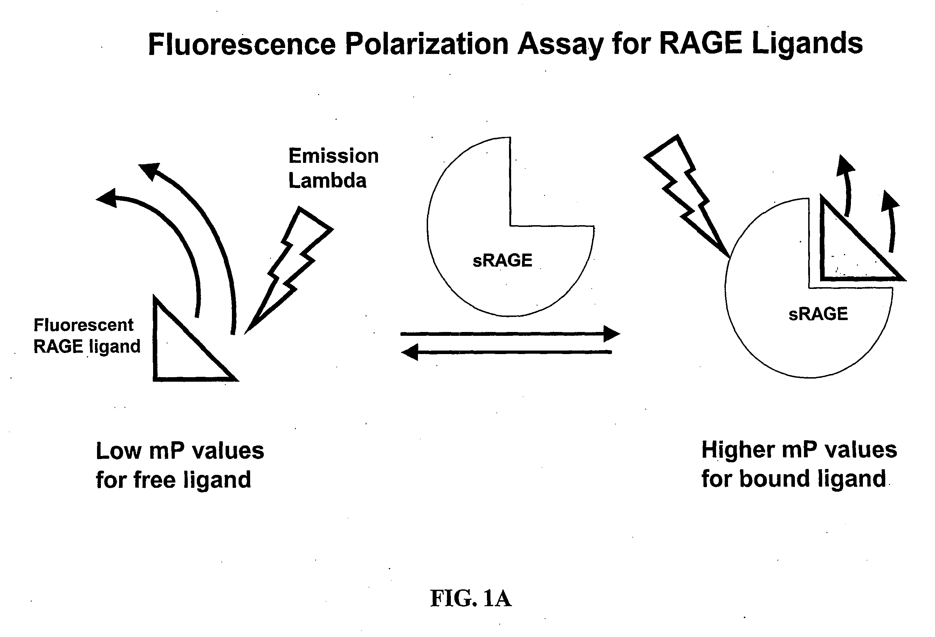

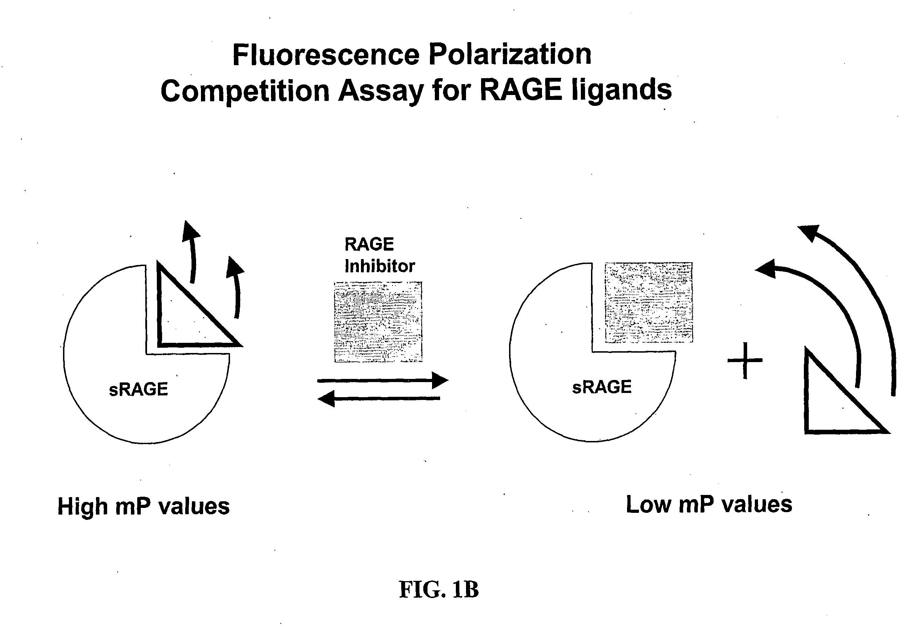

A schematic representation of a method and/or system of the present invention is shown as FIG. 1. Florescence polarization detection methods allow for quantification of the magnitude of receptor/ligand interactions. The physical process that allows fluorescence polarization detection of binding is based on the principle that smaller molecules rotate faster than larger molecules in solution. The greater rotation for smaller molecules translates into a smaller fluorescence polarization signal as compared to larger molecules in the same solution phase. Therefore, a relatively small ligand, such as a fluorescent derivative of amyloid beta 1-40 depicted schematically as the triangular structure in FIG. 1, will have a lower millipolarization (mP) value when it is free in solution, as compared to the milli-polarization value when the fluorescent ligand is bound to its cognate receptor (e.g., sRAGE, FIG. 1A). In one embodiment, the extent of the polarization may increase in a substantially linear manner as a function of the extent of the binding between ligand and receptor (Allen et al., J. Biomol Screen, 2:63-69 (2000)).

As shown in FIG. 1B, the assay may allow for evaluation of the ability of a non-labeled compound of interest (*e.g*., square structure) to displace the fluorescent ligand (triangular structure) from the RAGE ligand binding site. As the fluorescent ligand is displaced, the milli-polarization (mP) values will decrease as the average size of the structure containing the fluorescent label decreases, since the smaller unbound fluorescent ligand will rotate faster than the fluorescent ligand bound to the RAGE polypeptide (e.g., sRAGE).

In one embodiment, there are minimal filtration, wash, or transfer steps required to perform the assay. For example, to perform the assay, the reagents (i.e., fluorescent RAGE ligand; RAGE polypeptide; compound of interest; and assay buffer) may be added to a microplate well. After an incubation step, fluorescence may be measured using a fluorescent polarization reader. Thus, the simple methodology may provide a useful format for high-throughput drug screening.

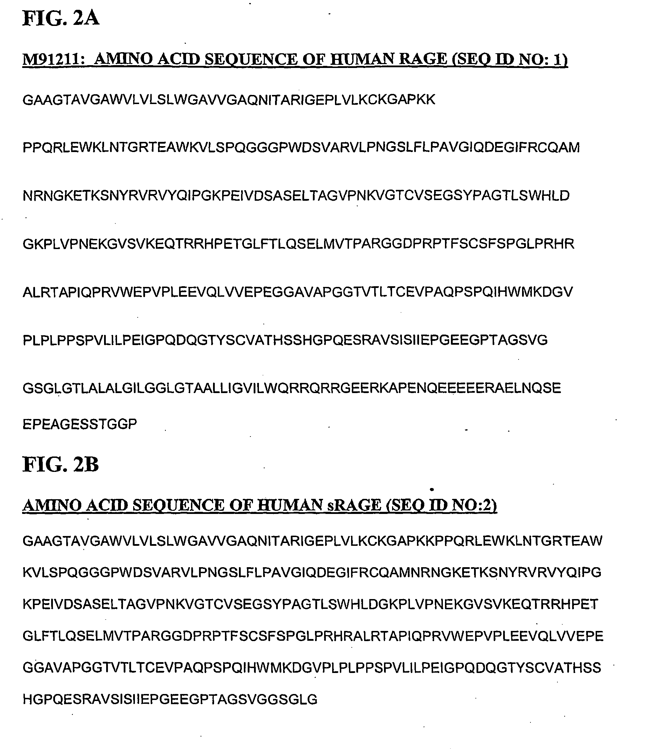

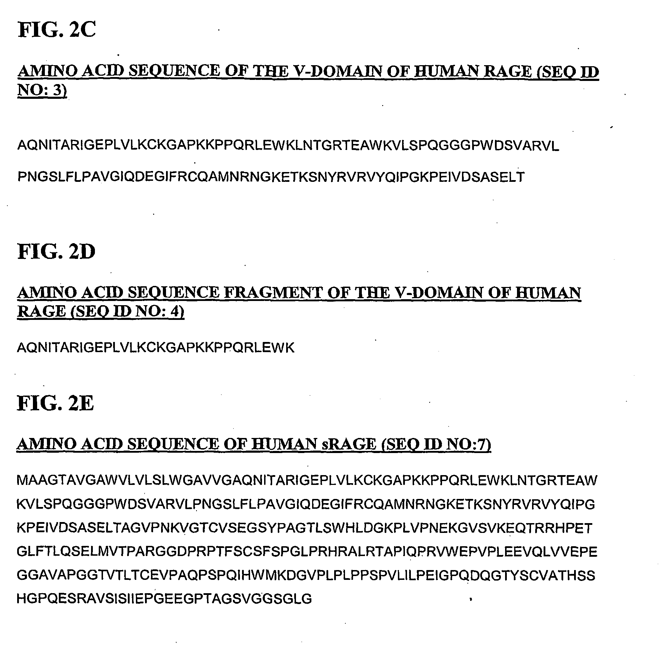

As described herein, a full-length RAGE protein or a fragment of the full-length RAGE protein may comprise the RAGE polypeptide used in the assay. Thus, a RAGE protein or polypeptide comprising a RAGE ligand binding domain may be the amino acid sequence shown as SEQ ID NO: 1 (FIG. 2A) or a portion of that amino acid sequence . (Neeper et al., J Biol. Chem. 267:14998-15004, (1992)). The ligand binding domain of RAGE comprises that region of the protein which is able to bind ligands with physiological specificity. As used herein, a fragment of the full-length RAGE protein is at least 5 amino acids in length, and may be greater than 30 amino acids in length, but is less than the full amino acid sequence. In one embodiment, the RAGE polypeptide comprises sRAGE (SEQ ID NO: 2; FIG. 2B), or a fragment thereof, wherein sRAGE is the RAGE protein free from the cell membrane (Park et al., Nature Med., 4:1025-1031 (1998)). In one embodiment, a sequence at least 90% identical to SEQ ID NO: 1 or SEQ ID NO: 2 may be used. For example, the RAGE polypeptide may comprise SEQ ID NO: 1 or SEQ ID NO: 2 but with methionine (M) as the first amino acid rather than glycine (G). For example, a recombinant form of sRAGE comprising Methionine (M) as the first residue (e.g., SEQ ID NO: 7; FIG. 2E) may be used. In another embodiment, the RAGE protein comprises the V domain (SEQ ID NO: 3; FIG. 2C) (Neeper et al., J. Biol. Chem. 267:14998-15004 (1992)), or a fragment thereof (*e.g*., SEQ ID NO: 4, FIG. 2D). In yet another embodiment, the RAGE fragment is a synthetic peptide.

The assay may be designed to allow for selection of an amount of RAGE polypeptide which comprises a linear region of binding of RAGE to the fluorescent ligand. For example, if too low of a concentration of RAGE polypeptide is employed, the detection of binding may be difficult due to low signal. If excess RAGE polypeptide is used, however, the ability of a compound of interest to compete with the fluorescent ligand may be lessened due to the excess RAGE polypeptide binding to both the ligand and the compound of interest. In alternate embodiments, the polypeptide comprising a RAGE ligand binding site may range from about 0.001 nM to about 50 µM, or from about 0.01 nM to about 5 µM final assay concentration (FAC), or from about 0.1 nM to about 500 nM FAC, or from about 1 nM to about 100 nM FAC. For each and every range described herein, it is contemplated that any range within the range may be used and thus, is described. Thus, a range comprising 1 nM to about 100 nM includes ranges such as, but not limited to, 2 nM to 99 nM, or 3 nM to 98 nM, and each and every range in between.

Also in an embodiment, the assay is designed to allow optimization of the fluorescent ligand. Thus, in an embodiment, the amount of fluorescent ligand used can be titered to allow for maximal signal, but is not so high as to comprise high levels of background binding. More preferably, the amount of fluorescent ligand allowing for maximum signal comprises a similar range regardless of the concentration of competing compound. In alternate embodiments, the final assay concentration of fluorescent ligand (e.g., fluorescent amyloid beta) may range from about 1 pM to about 50 µM, or from about 0.005 nM to about 5 µM, or from about 0.05 nM to about 500 nM, or from about 0.5 nM to 50 nM. Again, for each and every range described herein, it is contemplated that any range within the range may be used.

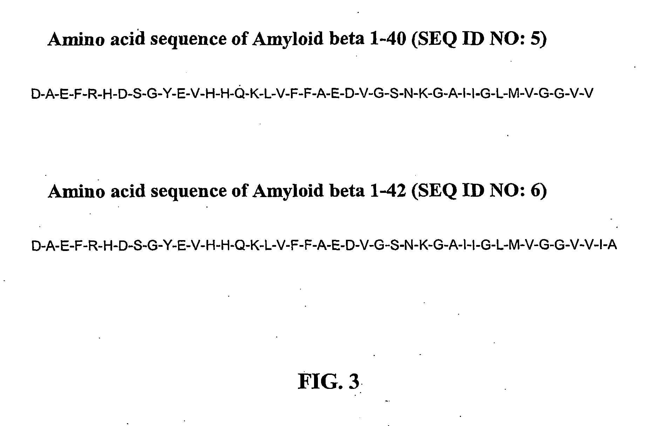

The fluorescent ligand comprises an amyloid beta polypeptide. For example, in alternate embodiments, the fluorescent ligand may comprise amyloid beta (1-40) having the amino acid sequence of SEQ ID NO: 5 or amyloid beta (1-42) having the amino acid sequence of SEQ ID NO: 6 (FIG. 3). In one embodiment, the fluorescein may be attached to the amino terminus of the amyloid beta polypeptide. Where amyloid beta is used as the labeled ligand, it may be necessary to preincubate the fluorescent amyloid beta before adding the ligand to the assay, as such preincubation may allow the amyloid beta to self-aggregate into pleated sheet form. In one embodiment, amyloid beta may preferentially bind to RAGE in the form of a pleated sheet.

The assay may also be optimized to test compounds of interest over a range of concentrations. In alternate embodiments, the compound of interest may be present in the assay at a concentration in the range of about 1 pM to 500 µM, or from about 0.010 nM to about 200 µM, or from about 0.1 nM to about 50 µM, or from about 1 nM to about 40 µM, or from about 10 nM to about 30 µM.

The methods and systems of the present invention may be designed to maximize assay sensitivity. For example, where sRAGE is used as the polypeptide comprising a RAGE binding site at a final assay concentration (FAC) of 40 nM, and the fluorescent ligand is amyloid beta (1-40), almost maximum signal may be seen with 50 nM amyloid beta (1-40). In one embodiment of the competition assay, concentrations between 10 nM to 30 µM unlabeled ligand (i.e., the compound of interest) per binding reaction well (40 µL assay volume) may comprise a linear decrease in the fluorescent ligand binding to sRAGE.

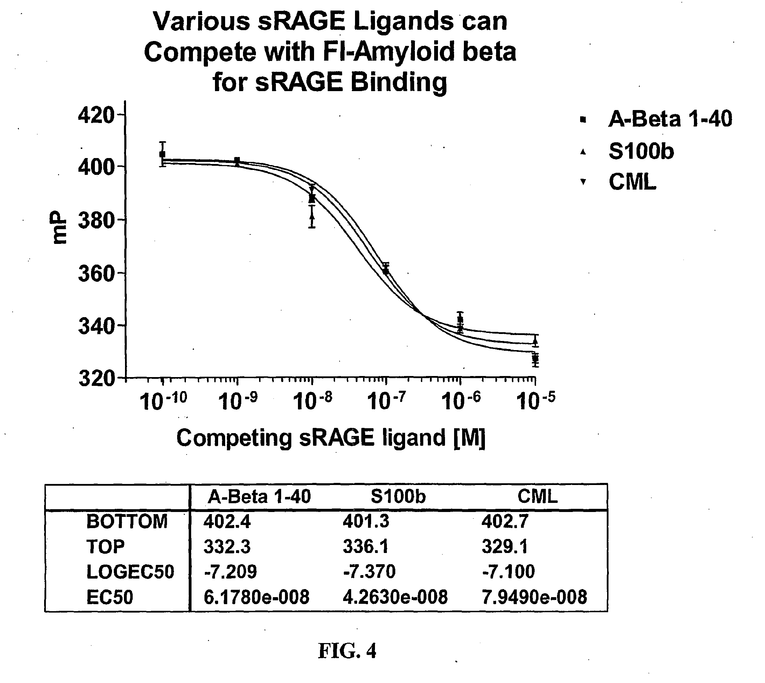

The assay may be used to detect and quantify the ability of a wide variety of potential RAGE modulators to displace a fluroceinated ligand from the binding domain of RAGE. As shown in FIG. 4, non-labeled RAGE ligands may compete for binding to sRAGE with an effective concentration (EC50) in a physiological range for the unlabeled ligand. For example, unlabeled amyloid beta (1-40) may compete with fluoresceinated amyloid beta (1-40) for binding to sRAGE with an EC50 of about 62 nM. Also, unlabeled s100b may compete with fluoresceinated amyloid beta (1-40) for binding to STAGE with an EC50 of about 43 nM. Also, unlabeled CML (carboxymethyllysine) may compete with fluoresceinated amyloid beta (1-40) for binding to sRAGE with an EC50 of about 79 nM. For the data of FIG. 4, the EC50 values are the concentrations of unlabeled ligand resulting in displacement of the fluorescent ligand halfway from the concentration at which maximal polarization results ("BOTTOM") to the concentration.resulting in minimum polarization ("TOP").

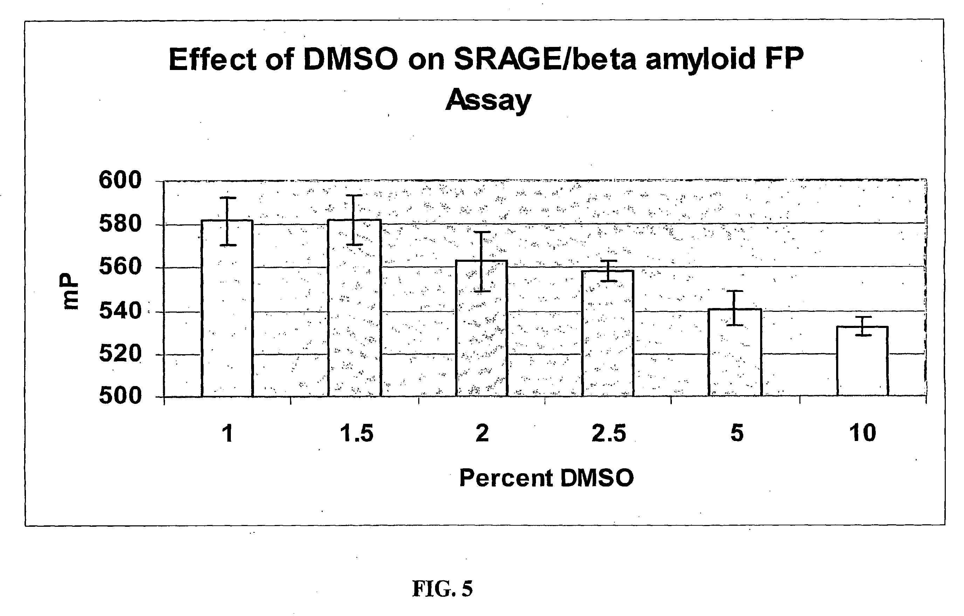

The assay may be optimized to be independent of any solvents that may be required for dissolving the compounds being tested. For example, in some cases, organic solvents such as dimethyl sulfoxide (DMSO) may be used to dissolve organic compounds that are to be tested as potential modulators of RAGE. For example, at a final assay concentration (FAC) of 1.5% DMSO, which is the concentration of DMSO generally employed for dissolution of organic compounds of interest in the assay, the assay may generally provide a maximal signal (FIG. 5). Even at concentrations as high as 10% DMSO, the assay may provide an adequate window for detection of fluoresceinated amyloid beta (1-40) binding to sRAGE and displacement of such binding by a compound of interest.

Thus, the assay may allow for optimization of each component, such that variation of assay results due to any experimental variation may minimized. Once optimized for a specific fluorescent ligand and RAGE polypeptide, the assay can provide a reliable assessment of sample binding affinity regardless of the specific competing compound of interest (*i.e.* putative modulator) which is being tested. Also, in an embodiment, the assay comprises a high-throughput assay that is reproducible and precise. In one embodiment, the assay comprises a variance of less than 20%. In alternate embodiments, the assay may comprise a variance of less than 10% or less than 5%.

The methods and systems of the present invention may be designed such that binding of the RAGE polypeptide to the fluorescent ligand will reflect the nature of the binding of RAGE to the ligand *in vivo.* For example, the fluorescent ligand may bind to the RAGE polypeptide in a specific and saturable manner. In one embodiment the assay may be designed such that an sRAGE polypeptide at concentrations ranging from about 0.1 nM to about 500 nM binds fluorescent amyloid beta (1-40) in a saturable manner. The methods and systems of the present invention may be designed to detect compounds that antagonize both low and high affinity binding sites. For example, using a RAGE polypeptide comprising a ligand binding domain, wherein the RAGE polypeptide is at concentrations that range from about 0.1 nM to about 10 nM, may provide for characterization of a high affinity binding site comprising a Kd of less than 10 nM using unlabeled amyloid beta (1-40) as the competing ligand. Also, using the RAGE polypeptide comprising a ligand binding domain at concentrations ranging from about 20 nM to about 500 nM may provide for characterization of a lower affinity binding site. Analysis of the data by means of Scatchard transformation may be used to show both high and low affinity sites (Hofmann et al., Cell, 97:889-901 (1999); Hori et al., J. Biol. Chem., 270:25752-25761 (1995)).

A premise of the assay is that small molecules rotate faster than large molecules and that the size of the molecule is reflected by the level of fluorescent polarization (i.e., mP values). By increasing the size of the complex comprising a RAGE ligand binding domain, there may be a larger change in polarization as a fluorescent ligand goes from a state where it is freely rotating in solution, to a state where the fluorescent ligand is bound to the RAGE ligand binding domain. Thus, in one embodiment, the polypeptide comprising a RAGE ligand binding domain may be complexed with a second polypeptide or protein as a way to increase the size of the receptor. For example, whereas binding of a fluorescent RAGE ligand to sRAGE may increase the millipolarization (mP) value (e.g., from 330 to 430), binding of the fluorescent RAGE ligand to sRAGE that is complexed with a second polypeptide may increase the polarization to even larger values.

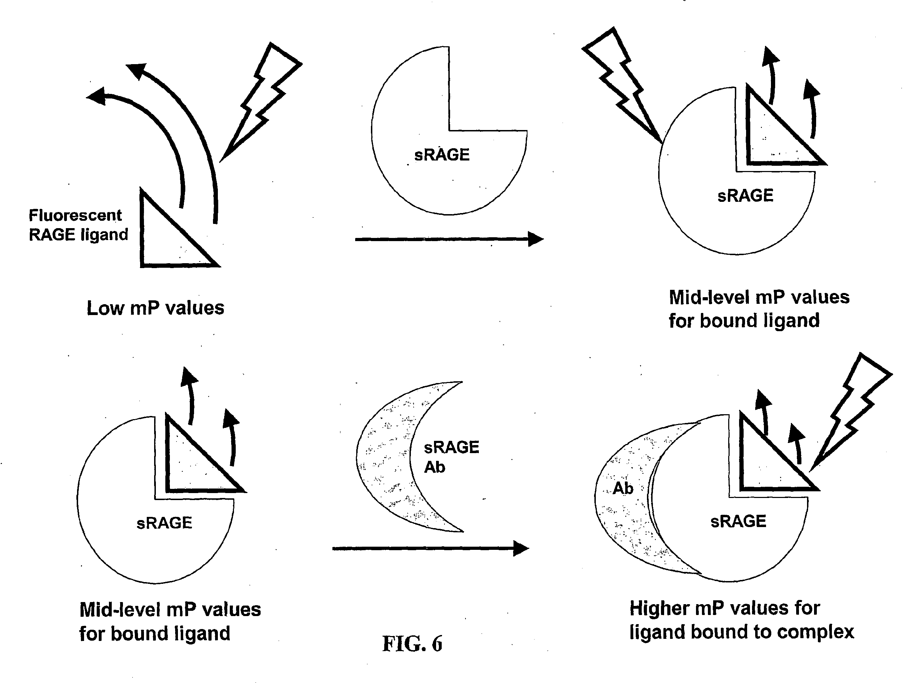

As shown in FIG. 6, the polypeptide comprising a RAGE ligand binding site may be allowed to complex with an anti-RAGE antibody. For example, STAGE may be used as the polypeptide comprising a RAGE ligand binding domain and anti-sRAGE antibody may be allowed to complex with the sRAGE. Antibodies that may be used for detection of sRAGE include commercially available anti-RAGE polyclonal antibodies (e.g., Goat Anti-RAGE polyclonal antibody, Cat. No.: ab7714; Novus Biologicals, Littleton, CO; Goat Anti-Human RAGE polyclonal antibody, Cat. No.: ST1025, CalBiochem, San Diego, CA) and monoclonal antibodies (Anti-Human RAGE monoclonal antibody, Cat. No.: R1031, United States Biological, Swampscott, MA). Also, custom-made anti-RAGE antibodies may be made as is known in the art.

The method used to form the complex between the polypeptide comprising the RAGE ligand binding domain and the antibody may depend upon the antibody used. It may be preferred that the antibody not interfere with, or modify, binding of the fluorescent RAGE ligand to the RAGE binding domain.

The assay buffer may include components to reduce background binding of the second polypeptide (e.g., sRAGE antibody) to the well. For example, the assay components may be prepared using a buffer that includes BSA (e.g., 0.5% to 0.5%) and/or TWEEN 20 (e.g., 0.01-0.1%). Alternatively, the assay well may be treated with a blocking buffer (e.g., 50 mM imidazole, pH 7.2, 1-5% bovine serum albumin (BSA), 0.01-0.1% TWEEN 20) to reduce background binding of the antibody to the assay well. The blocking buffer may then be aspirated from the wells, and the assay well washed. For example, after treatment of the assay well with a blocking buffer, the assay well may be washed three times with 400 µl/well with wash buffer (e.g., 20 mM imidazole, pH 7.2; 150 mM NaCl), with an optional soak in wash buffer between each wash.

To perform the assay, the individual components (e.g., 5-10 µl of the compound of interest dissolved in DMSO), sRAGE (e.g., 10 µl sRAGE, 40 nM FAC), fluorescent ligand (e.g., 5-10 µl fluorescent amyloid beta, 50 nM FAC) and antibody (e.g., 10 µl monoclonal antibody to sRAGE,) may then be added simultaneously to each well, and incubated 1 hour at 37°C. Alternatively, the antibody and the RAGE polypeptide may be allowed to incubate to form a complex prior to adding them to the assay well. The complex may be formed at 4°C to 37°C for a time period that may range from about 30 minutes to 24 hours. Generally, the warmer incubation temperatures will allow for a shorter incubation time. In yet another embodiment, the polypeptide comprising the binding domain of RAGE may be allowed to complex with an anti-RAGE antibody after the RAGE polypeptide is allowed to bind the fluorescent RAGE ligand and/or compound of interest. This approach may be preferred where it is desired to minimize any potential interference by the antibody of the binding of the fluorescent ligand and compound of interest to the RAGE polypeptide.

The assay may be designed to allow optimization of the amount of anti-RAGE antibody used. Thus, in an embodiment, the amount of antibody used can be titered to allow for maximal signal, but not so high as to result in high levels of background binding. The preferred amount of antibody may be based upon the amount of RAGE polypeptide used in the assay and the affinity of the antibody for RAGE. In one embodiment, the amount of antibody allowing for maximum signal may comprise a similar range regardless of the concentration of the compound of interest or the fluorescent RAGE ligand.

The polypeptide comprising the RAGE ligand binding domain may be prepared using recombinant techniques. As is known in the art, the nucleic acid constructs used to express RAGE polypeptides used in the methods and systems of the present invention may be modified by mutation, as for example, by PCR amplification of a nucleic acid template with primers comprising a mutation of interest. In this way, polypeptides comprising varying affinity for RAGE ligands may be designed. In one embodiment, the mutated sequences may be 90% or more identical to the starting DNA. Or the mutated sequences may be 80%, or 70% or more identical to the starting DNA. As such, variants may include nucleotide sequences that hybridize under stringent conditions (e.g., equivalent to about 20-27°C below the melting temperature (TM) of the DNA duplex in 1 molar salt).

The RAGE polypeptides and proteins used in the methods and systems of the present invention may be further modified for increased efficacy. Thus, the RAGE polypeptides used for the methods and systems of the present invention may be modified by post-translational processing or by chemical modification. For example, the RAGE polypeptide may be synthetically prepared to include L-, D-, or unnatural amino acids, alpha-disubstituted amino acids, or N-alkyl amino acids. Additionally, the RAGE polypeptide may be modified by acetylation, acylation, ADP-ribosylation, amidation, attachment of lipids such as phosphatidyinositol, formation of disulfide bonds, and the like.

The methods and systems of the present invention provide binding assays to identify compounds that interact with RAGE under physiological binding conditions. In this respect, physiological binding conditions comprise those conditions which result in binding affinities similar to those seen *in vivo.*

Thus, in another embodiment, the present specification comprises a compound identified by the methods and/or systems of the present invention as having the ability to modulate ligand binding to RAGE. Compounds identified by the methods and/or systems of the invention may comprise several different chemical types. For example, in one embodiment, the RAGE modulator compound may comprise a peptide. In another embodiment, the RAGE modulator compound may comprise a peptidomimetic. In another embodiment, the RAGE modulator compound may comprise an organic molecule. In yet another embodiment, the RAGE modulator compound may comprise an inorganic molecule. In some cases, the RAGE modulator compound may be derivatized to increase its half-life.

As described above, the compound identified as having the ability to modulate RAGE may comprise a peptidomimetic. In an embodiment, the peptidomimetic is at least partly unnatural. Preferably the compound of interest may be modified to increase stability, efficacy, potency and bioavailability. For example, in an embodiment, the compound may be synthetically prepared to include L-, D-, or unnatural amino acids, alpha-disubstituted amino acids, N-alkyl amino acids, or lactic acid. In an embodiment, the compound comprises a peptidomimetic having a peptide backbone or amino acid replaced with a suitable mimetic.

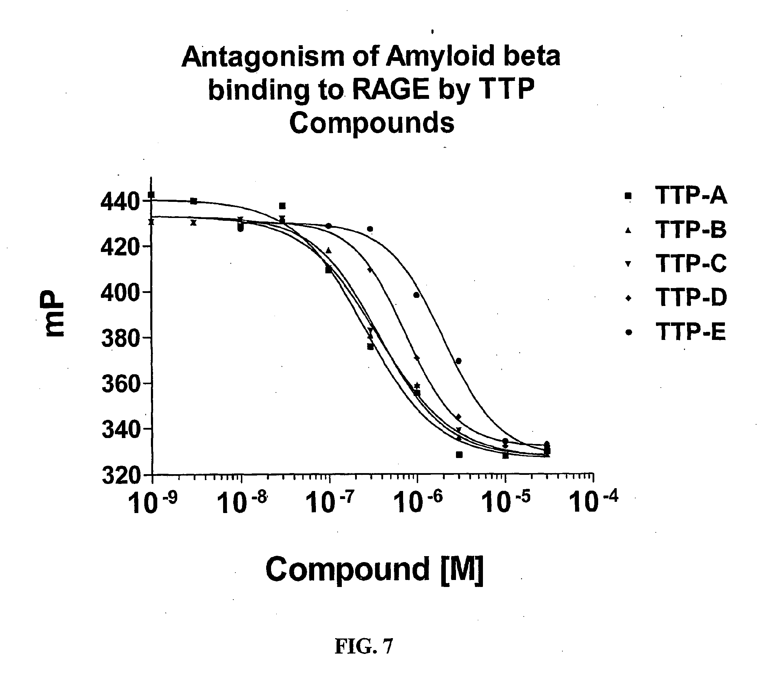

Also, the compound identified as having the ability to modulate RAGE may comprise a small organic molecule. In an embodiment, the organic molecule comprises a molecular weight of less than 1000 Daltons. For example, as shown in FIG. 7, TTP-A, TTP-B, TTP-C, TTP-D, and TTP-E inhibit sRAGE binding to fluorescent amyloid beta (1-40) with IC50 values of about 259 nM, 376 nM, 367 nM, 749 nM and 2.04 µM respectively. The structure of small organic compounds of interest such as, and including TTP-A, TTP-B, TTP-C, TTP-D, and TTP-E, are provided in commonly owned U.S. Patent Applications having Publication Nos. 2002/0006957, 2003/0032663, 2002/0193432, and 2004/0082542.

In an embodiment, compounds identified by the binding assay are further tested as having the ability to modulate a biological activity of RAGE. For example, compounds can be tested for their ability to modulate RAGE-induced increases in gene expression or other cellular processes. Thus, in an embodiment, compounds identified by the binding assay may be used to modulate RAGE activation of NF-κB mediated transcription. The ability of the potential modulator compound to modulate RAGE activation of NF-κB mediated transcription may be assessed using a reporter gene downstream of an NF-κB promoter. Alternatively, compounds identified by the binding assay may be used to modulate RAGE activation of TNF-α mediated transcription, where the ability of the potential modulator compound to modulate RAGE activation of TNF-α mediated transcription may be assessed using a reporter gene downstream of an TNF-α promoter. In one embodiment, compounds which modulate ligand binding to RAGE will be identified as modulating the effects of RAGE on other cellular processes.

### EXAMPLES

Features and advantages of the inventive concept covered by the present invention are further illustrated in the examples which follow.

### Example 1: Fluorescent Polarization Assay Protocol

Breifely, the method involves adding 10 uL of recombinant human sRAGE (final assay concentration 40 nM having the amino acid sequence SEQ ID NO: 7) to 10 uL of a phosphate buffered saline pH 7.4 (Sigma, St.Louis MO) and then adding 10 uL (final assay concentration 50 nM) of the fluorescent amyloid beta (1-40) peptide (Biosource, Camarillo, CA) in the presence of 10 uL of RAGE antagonists (final assay concentrations 10 nM to 30 uM). It may necessary to preincubate the fluorescent amyloid beta ligand so that the amyloid beta forms a pleated sheet structure since amyloid beta that is not in a pleated sheet structure may not bind to sRAGE with high affinity. To form a pleated sheet structure, the amyloid beta may be preincubated for at least 24 hrs prior to being used in the assay. To preincubate the amyloid beta, the lyophilized peptide is dissolved in high pressure liquid chromatography (HPLC) grade water at a concentration of 6 mg/ml. Once dissolved, the peptide is diluted with phosphate-buffered saline (calcium-free) to a concentration of 1 mg/ml and allowed to incubate at 37°C for 24-48 hours.

The complex was incubated at 37°C for 1 hour and then the milli-polarization (mP) value was read using an Envision plate reader (Perkin Ekmer). In some cases, depending on the purity of the human sRAGE, 0.1% bovine serum albumin (BSA) or 0.05% of the detergent TWEEN 20 may be added to the phospahte buffered saline to reduce background binding of the sRAGE to the assay well.

### Example 2: Optimization of Assay Conditions

Experiments for optimization of assay conditions were performed. For example, using the recombinant sRAGE at a final assay concentration of 40 nM, it was found that fluorescent amyloid beta concentrations between 4 to 400 ng per ml per 40 µL reaction volume resulted in a linear increase in sRAGE binding.

Next, optimization of the assay at varying sRAGE concentrations was evaluated. Using 50 nM fluorescent labeled amyloid beta (1-40), it was found that binding ofsRAGE increased in a linear manner at concentrations ranging from 0.6 to 600 ng per ml sRAGE per 40 µL assay volume, with a maximum signal seen at about 40 nM sRAGE.

A representative experiment showing the effect of increasing concentrations of DMSO on the milli-polarization (mP) value for sRAGE interacting with the fluorescienated amyloid beta 1-40 ligand is shown as FIG.5. DMSO concentrations of up to 2% final volume had no significant effect on the milli-polarization values obtained. Although there was some effect on milli-polarization values at DMSO concentrations above 2% FAC (final assay concentration), a window was achieved with DMSO concentrations above 2% as the baseline mP value (fluorescienated amyloid beta 1-40 ligand with no sRAGE present) was about 470±5 mP, giving an adequate window of detection. Each bar represents the mean of four measurements with a calculated standard deviation

Generally, the assays employed 0.5 nM to 50 nM fluorescent amyloid beta and 1 to 100 nM sRAGE. Compounds of interest ranged from 1 nM to 30 µM.

Experiments were performed to quantify assay variability. It was found that generally, assay variability was less than 10%.

### Example 3: Detection of Ligand Binding to sRAGE.

FIG. 4 shows a representative experiment which demonstrates that RAGE specific ligands compete with the fluorescienated amyloid beta 1-40 for binding to sRAGE. The competition occurs in a dose dependent manner where increasing amounts of the unlabeled ligands displaced the fluorescent amyloid beta 1-40 ligand, resulting in a lower millipolarization (mP) value.

The EC50 values of 62 nM for unlabeled amyloid beta 1-40 (squares), 43 nM for unlabeled S100b (triangle), and 79 nM for unlabeled Carboxymethyllysine (inverted triangle) agree with literature values for binding to sRAGE (Yan et al., Nature, 382:685-691 (1996), Kislinger et al., J. Biol. Chem. 274: 31740-31749 (1999)). Note that each point represents the mean of four measurements with a calculated standard deviation.

### Example 4: Inhibition of sRAGE Binding by Small Organic Modulators

Representative experiments that demonstrate that a small organic molecules can compete with the fluorescienated amyloid beta 1-40 for binding to sRAGE are shown as FIG. 7. For example, TTP-A, TTP-B, TTP-C, TTP-D, and TTP-E inhibit sRAGE binding to fluorescent amyloid beta (1-40) with IC50 values of about 259 nM, 376 nM, 367 nM, 749 nM and 2.04 µM respectively. The competition occurs in a dose dependent manner where increasing amounts of the unlabeled ligand displaces increasing amounts of the fluorescent amyloid beta 1-40 ligand, resulting in a lower milli-polarization (mP) value. The concentration of unlabeled ligand that results in 50% displacement of the fluorescent amyloid beta is the IC₅₀ value. This data demonstrates that the assay of the present invention can be utilized as a screen for small organic molecules for RAGE antagonist activity. In FIG. 7, each point represents the mean of four measurements with a calculated standard deviation.

## Claims

**1.** A method for detection of RAGE (Receptor for Advanced Glycated Endproducts) modulators comprising:
(a) providing (i) a RAGE polypeptide comprising a ligand binding domain of RAGE, (ii) a fluorescent RAGE ligand, wherein the fluorescent RAGE ligand comprises an amyloid beta polypeptide, and (iii) a compound of interest;
(b) adding the compound of interest and the fluorescent RAGE ligand to the RAGE polypeptide; and
(c) measuring the polarization of the fluorescent RAGE ligand; and
(d) correlating the level of polarization to the amount of the fluorescent RAGE ligand that is bound to the RAGE polypeptide.

**2.** The method of claim 1, further comprising measuring the amount of fluorescent RAGE ligand bound to the RAGE polypeptide in the presence of varying amounts of the compound of interest.

**3.** The method of claim 2, further comprising determining the affinity of the compound of interest for the RAGE ligand binding domain based on the amount of the compound of interest required to reduce the binding of the fluorescent RAGE ligand to the RAGE polypeptide by a predetermined amout,

**4.** The method of claim 3, wherein the ability of the compound of interest to reduce binding of the fluorescent RAGE ligand to the RAGE polypeptides is compared to the ability of a second compound to reduce binding of the fluorescent RAGE ligand to the RAGE polypeptide.

**5.** The method of claim 4, wherein the second compound does not substantially bind to the RAGE ligand binding domain, such that the second compound functions as a negative control for the measurement of binding to the RAGE ligand binding domain.

**6.** The method of claim 4, wherein the second compound comprises a substantial binding affinity for the RAGE ligand binding domain, such that the second compound functions as a positive control for the measurement of binding to the RAGE ligand binding domain.

**7.** The method of claim 4, wherein the second compounds comprises at least one of a structural variant or chemical variant of the compound of interest.

**8.** The method of claim 1, wherein the fluorescent RAGE ligand is preincubated to promote self-aggregation of the amyloid beta polypeptide into a pleated sheet form

**9.** The method of claim 1, wherein the amlyoid beta peptide comprises amyloid beta (1-40) having the amino acid sequence as set forth in SEQ ID NO: 5, or
amyloid beta (1-42) having the amino acid sequence as set forth in SEQ ID NO: 6.

**10.** The method of claim 1, wherein a fluorescein moiety is attached to the amino terminus of the amyloid beta polypeptide.

**11.** The method of claim 1, wherein the polypeptide comprising a ligand binding domain of RAGE comprises the amino acid sequence as set forth in SEQ ID NO: 4, SEQ ID NO: 3, or SEQ ID NO: 7, or a sequence at least 90% identical thereto.

**12.** The method of claim 1, wherein the polypeptide comprising a ligand binding domain of RAGE is covalently linked to, or allowed to complex with, a second polypeptide.

**13.** The method of claim 12, wherein the polypeptide comprising a ligand binding domain of RAGE is allowed to complex with an antibody that recognizes RAGE or a fragment thereof.

**14.** The method of claim 1, wherein the compound of interest comprises at least one of an organic molecule, a peptide, a peptidomimetic, an inorganic compound, a lipid, a carbohydrate, or a nucleic acid.

**15.** The method of claim 14, wherein the small organic molecule has a molecular weight of less than 1000 Daltons.

**16.** The method of claim 1, wherein the compound of interest is a RAGE antagonist.

**17.** The method of claim 1, wherein the compound of interest is a RAGE agonist.

**18.** The method of claim 1, wherein the compound of interest binds to the ligand binding domain of the RAGE polypeptide,

**19.** The method of claim 1, wherein the concentration of the fluorescent ligand in the assay ranges from 1 pM to 50 µM, or from 0.005 nM to 5 µM, or from 0.05 nM to 500 nM, or from 0.5 nM to 50 nM.

**20.** The method of claim 1, wherein the concentration of the polypeptide comprising a RAGE ligand binding site in the assay ranges from 0,001 nM to 50 µM, or from 0.01 nM to 5 µM, or from 0.1 nM to 500 nM, or from 1 nM to 100 nM.

**21.** The method of claim 5, wherein the negative control exhibits a dissociation constant (Kd) for binding to the RAGE ligand binding domain of greater than 5 µM, or greater than 10 µM, or greater than 50 µM, or greater than 100 µM, or greater than ImM.

**22.** The method of claim 6, wherein the positive control exhibits a dissociation constant (Kd) for binding to the RAGE ligand binding domain of less than 1 µM, or less than 200 nM, or less than 50 nM, or less than 10 nM.

**23.** A system for detection of compounds that modulate the binding of a ligand to RAGE using the methods of claims 1-22 comprising individually packaged containers of:
(a) a fluorescent RAGE ligand, wherein the fluorescent ligand comprises an amyloid beta polypeptide;
(b) a RAGE polypeptide comprising a ligand binding domain of RAGE,; and
(c) at least one assay reagent for dilution of (a) and (b) in the presence of a compound of interest, such that binding of the fluorescent RAGE ligand to the polypeptide comprising the ligand binding domain of RAGE may be quantified.

**24.** The system of claim 23, further comprising a device to measure the polarization of the fluorescent ligand.

**25.** The system of claim 23, further comprising a compound comprising a predetermined binding affinity for the RAGE ligand binding domain.

**26.** The system of claim 25, wherein the compound comprising a predetermined binding affinity for the RAGE ligand binding domain does not substantially bind to the RAGE binding domain, such that the second compound functions as a negative control for the measurement of binding to the RAGE ligand binding domain.

**27.** The system of claim 25, wherein the second compounds comprises a substantial binding affinity for the RAGE binding domain, such that the second compound functions as a positive control for the measurement of binding to the RAGE ligand binding domain.

**28.** The system of claim 23, further comprising a second polypeptide that is linked to or can complex with the RAGE polypeptide.

**29.** The system of claim 28, wherein the second polypeptide comprises an antibody that recognizes RAGE or a fragment of RAGE.

## Patentansprüche

1. Verfahren zur Detektion von RAGE(Receptor for Advanced Glycated Endproducts)-Modulatoren, umfassend:
(a) Bereitstellen (i) eines RAGE-Polypeptids, das eine Ligandenbindungsdomäne von RAGE umfasst, (ii) eines fluoreszierenden RAGE-Liganden, wobei der fluoreszierende RAGE-Ligand ein Beta-Amyloidpolypeptid umfasst, und (iii) einer interessierenden Verbindung,
(b) Hinzugeben der interessierenden Verbindung und des fluoreszierenden RAGE-Liganden zum RAGE-Polypeptid und
(c) Messen der Polarisation des fluoreszierenden RAGE-Liganden und
(d) Korrelieren des Polarisationsniveaus mit der Menge an fluoreszierendem RAGE-Liganden, der an das RAGE-Polypeptid gebunden ist.

2. Verfahren nach Anspruch 1, weiter umfassend Messen der Menge an fluoreszierendem RAGE-Liganden, der an das RAGE-Polypeptid gebunden ist, in Gegenwart verschiedener Mengen an interessierender Verbindung.

3. Verfahren nach Anspruch 2, weiter umfassend Bestimmen der Affinität der interessierenden Verbindung zu der RAGE-Ligandenbindungsdomäne basierend auf der Menge an interessierender Verbindung, die erforderlich ist, um die Bindung des fluoreszierenden RAGE-Liganden an das RAGE-Polypeptid um eine vorgegebene Menge zu verringern.

4. Verfahren nach Anspruch 3, wobei die Fähigkeit der interessierenden Verbindung, die Bindung des fluoreszierenden RAGE-Liganden an das RAGE-Polypeptid zu verringern, mit der Fähigkeit einer zweiten Verbindung die Bindung des fluoreszierenden RAGE-Liganden an das RAGE-Polypeptid zu verringern verglichen wird.

5. Verfahren nach Anspruch 4, wobei die zweite Verbindung nicht wesentlich an die RAGE-Ligandenbindungsdomäne bindet, so dass die zweite Verbindung als negative Kontrolle für die Messung einer Bindung der RAGE-Ligandenbindungsdomäne dient.

6. Verfahren nach Anspruch 4, wobei die zweite Verbindung eine wesentliche Bindungsaffinität für die RAGE-Ligandenbindungsdomäne aufweist, so dass die zweite Verbindung als positive Kontrolle für die Messung einer Bindung der RAGE-Ligandenbindungsdomäne dient.

7. Verfahren nach Anspruch 4, wobei die zweite Verbindung eine strukturelle Variante und/oder eine chemische Variante der interessierenden Verbindung umfasst.

8. Verfahren nach Anspruch 1, wobei der fluoreszierende RAGE-Ligand so vorinkubiert wird, dass er eine Selbstaggregation dies Beta-Amyloidpolypeptids in eine Faltblattform begünstigt.

9. Verfahren nach Anspruch 1, wobei das Beta-Amyloidpolypeptid Beta(1-40)-Amyloid, das die in SEQ ID NO:5 angegebene Aminosäuresequenz aufweist, oder Beta(1-42)-Amyloid umfasst, das die in SEQ ID NO:6 angegebene Aminosäuresequenz aufweist.

10. Verfahren nach Anspruch 1, wobei eine Fluoreszenzgruppe mit der Aminoendstelle des Beta-Amyloidpolypeptids verknüpft wird.

11. Verfahren nach Anspruch 1, wobei das Polypeptid, das eine Ligandenbindungsdomäne von RAGE umfasst, die die in SEQ ID NO:4, SEQ ID NO:3 oder SEQ ID NO:7 angegebene Aminosäuresequenz oder eine mindestens zu 90 % damit identische Sequenz aufweist.

12. Verfahren nach Anspruch 1, wobei das Polypeptid, das eine Ligandenbindungsdomäne von RAGE umfasst, mit einem zweiten Polypeptid kovalent verknüpft wird oder damit komplexiert.

13. Verfahren nach Anspruch 12, wobei das Polypeptid, das eine Ligandenbindungsdomäne von RAGE umfasst, mit einem Antikörper komplexiert, der RAGE oder ein Fragment davon erkennt.

14. Verfahren nach Anspruch 1, wobei die interessierende Verbindung ein organisches Molekül, ein Peptid, ein Peptidomimetikum, eine anorganische Verbindung, ein Lipid, ein Kohlenhydrat und/oder eine Nukleinsäure umfasst.

15. Verfahren nach Anspruch 14, wobei das kleine organische Molekül ein Molekulargewicht von weniger als 1000 Dalton aufweist.

16. Verfahren nach Anspruch 1, wobei die interessierende Verbindung ein RAGE-Antagonist ist.

17. Verfahren nach Anspruch 1, wobei die interessierende Verbindung ein RAGE-Agonist ist.

18. Verfahren nach Anspruch 1, wobei die interessierende Verbindung an die Ligandenbindungsdomäne des RAGE-Polypeptids bindet.

19. Verfahren nach Anspruch 1, wobei die Konzentration des fluoreszierenden Liganden im Assay im Bereich von 1 pM bis 50 µM, oder von 0,005 nM bis 5 µM, oder von 0,05 nM bis 500 nM, oder von 0,5 nM bis 50 nM liegt.

20. Verfahren nach Anspruch 1, wobei die Konzentration des Polypeptids, das eine RAGE-Ligandenbindungsstelle umfasst, im Assay im Bereich von 0,001 nM bis 50 µM, oder von 0,01 nM bis 5 µM, oder von 0,1 nM bis 500 nM, oder von 1 nM bis 100 nM liegt.

21. Verfahren nach Anspruch 5, wobei die negative Kontrolle eine Dissoziationskonstante (Kd) zur Bindung an die RAGE-Ligandenbindungsdomäne von mehr als 5 µM, oder mehr als 10 µM, oder mehr als 50 µM, oder mehr als 100 µM, oder mehr als 1 mM zeigt.

22. Verfahren nach Anspruch 6, wobei die positive Kontrolle eine Dissoziationskonstante (Kd) zur Bindung an die RAGE-Ligandenbindungsdomäne von weniger als 1 µM, oder weniger als 200 nM, oder weniger als 50 nM, oder weniger als 10 nM zeigt.

23. System zur Detektion von Verbindungen, die die Bindung eines Liganden an RAGE modulieren, unter Verwendung der Verfahren nach den Ansprüchen 1 bis 22, umfassend einzeln verpackte Behälter mit:
(a) einem fluoreszierenden RAGE-Liganden, wobei der fluoreszierende Ligand ein Beta-Amyloidpolypeptid umfasst,
(b) einem RAGE-Polypeptid, das eine Ligandenbindungsdomäne von RAGE umfasst, und
(c) mindestens einem Assayreagens zur Verdünnung von (a) und (b) in Gegenwart einer interessierenden Verbindung, so dass eine Bindung des fluoreszierenden RAGE-Liganden an das Polypeptid, das die Ligandenbindungsdomäne von RAGE umfasst, quantitativ bestimmt werden kann.

24. System nach Anspruch 23, weiter umfassend eine Vorrichtung zum Messen der Polarisation des fluoreszierenden Liganden.

25. System nach Anspruch 23, weiter umfassend eine Verbindung, die eine vorgegebene Bindungsaffinität für die RAGE-Ligandenbindungsdomäne aufweist.

26. System nach Anspruch 25, wobei die Verbindung, die eine vorgegebene Bindungsaffinität für die RAGE-Ligandenbindungsdomäne aufweist, nicht wesentlich an die RAGE-Ligandenbindungsdomäne bindet, so dass die zweite Verbindung als negative Kontrolle für die Messung einer Bindung an die RAGE-Ligandenbindungsdomäne dient.

27. System nach Anspruch 25, wobei die zweite Verbindung eine wesentliche Bindungsaffinität für die RAGE-Bindungsdomäne aufweist, so dass die zweite Verbindung als positive Kontrolle für die Messung einer Bindung an die RAGE-Ligandenbindungsdomäne dient.

28. System nach Anspruch 23, weiter umfassend ein zweites Polypeptid, das mit dem RAGE-Polypeptid verknüpft wird oder damit komplexieren kann.

29. System nach Anspruch 28, wobei das zweite Polypeptid einen Antikörper umfasst, der RAGE oder ein Fragment von RAGE erkennt.

## Revendications

1. Procédé de détection de modulateurs du récepteur des produits de glycation avancée, RAGE (Receptor for Advanced Glycated End products), comprenant les étapes consistant à :
(a) mettre à disposition (i) un polypeptide RAGE comprenant un domaine de liaison à un ligand de RAGE, (ii) un ligand de RAGE fluorescent, où le ligand de RAGE fluorescent comprend un polypeptide bêta-amyloïde, et (iii) un composé d'intérêt ;
(b) ajouter le composé d'intérêt et le ligand de RAGE fluorescent au polypeptide RAGE ; et
(c) mesurer la polarisation du ligand de RAGE fluorescent ; et
(d) corréler le niveau de polarisation à la quantité de ligand de RAGE fluorescent qui est lié au polypeptide RAGE.

2. Procédé selon la revendication 1, comprenant en outre l'étape consistant à mesurer la quantité de ligand de RAGE fluorescent lié au polypeptide RAGE en présence de quantités variables du composé d'intérêt.

3. Procédé selon la revendication 2, comprenant l'étape consistant en outre à déterminer l'affinité du composé d'intérêt pour le domaine de liaison à un ligand de RAGE, en se basant sur la quantité du composé d'intérêt nécessaire pour réduire la liaison du ligand de RAGE fluorescent au polypeptide RAGE par une quantité prédéterminée.

4. Procédé selon la revendication 3, où l'aptitude du composé d'intérêt à réduire la liaison du ligand de RAGE fluorescent au polypeptide RAGE est comparée à l'aptitude d'un second composé à réduire la liaison du ligand de RAGE fluorescent au polypeptide RAGE.

5. Procédé selon la revendication 4, où le second composé ne se lie pas essentiellement au domaine de liaison à un ligand de RAGE, si bien que le second composé fonctionne comme un contrôle négatif pour la mesure de la liaison du domaine de liaison à un ligand de RAGE.

6. Procédé selon la revendication 4, où le second composé comprend une affinité de liaison substantielle pour le domaine de liaison à un ligand de RAGE, si bien que le second composé fonctionne comme un contrôle positif pour la mesure de la liaison du domaine de liaison à un ligand de RAGE.

7. Procédé selon la revendication 4, où le second composé comprend au moins un variant structurel ou un variant chimique du composé d'intérêt.

8. Procédé selon la revendication 1, où le ligand de RAGE fluorescent est préincubé pour favoriser une auto-agrégation du polypeptide bêta-amyloïde sous une forme de feuillet plissé.

9. Procédé selon la revendication 1, où le peptide beta-amyloïde consiste en bêta-amyloïde(1-40) ayant la séquence d'acides aminés telle que décrite dans SEQ ID NO: 5, ou
bêta-amyloïde(1-42) ayant la séquence d'acides aminés telle que décrite dans SEQ ID NO: 6.

10. Procédé selon la revendication 1, où un fragment fluorescéine est attaché à la terminaison amino du polypeptide bêta-amyloïde.

11. Procédé selon la revendication 1, où le polypeptide comprenant un domaine de liaison à un ligand de RAGE comprend la séquence d'acides aminés telle que décrite dans SEQ ID NO: 4, SEQ ID NO: 3, ou SEQ ID NO: 7, ou une séquence au moins 90 % identique à celle-ci.

12. Procédé selon la revendication 1, où le polypeptide comprenant un domaine de liaison à un ligand de RAGE est lié de façon covalente à, ou peut former un complexe avec, un second polypeptide.

13. Procédé selon la revendication 12, où le polypeptide comprenant un domaine de liaison à un ligand de RAGE peut former un complexe avec un anticorps qui reconnaît RAGE ou un fragment de celui-ci.

14. Procédé selon la revendication 1, où le composé d'intérêt comprend au moins un élément parmi une molécule organique, un peptide, un peptidomimétique, un composé inorganique, un lipide, un glucide, ou un acide nucléique.

15. Procédé selon la revendication 14, où la petite molécule organique possède un poids moléculaire inférieur à 1000 Daltons.

16. Procédé selon la revendication 1, où le composé d'intérêt est un antagoniste de RAGE.

17. Procédé selon la revendication 1, où le composé d'intérêt est un agoniste de RAGE.

18. Procédé selon la revendication 1, où le composé d'intérêt se lie au domaine de liaison à un ligand du polypeptide RAGE.

19. Procédé selon la revendication 1, où la concentration du ligand fluorescent dans l'essai est comprise entre 1 pM et 50 µM, ou entre 0,005 nM et 5 µM, ou entre 0,05 nM et 500 nM, ou entre 0,5 nM et 50 nM.

20. Procédé selon la revendication 1, où la concentration du polypeptide comprenant un site de liaison à un ligand de RAGE dans l'essai est comprise entre 0,00 nM et 50 µM, ou entre 0,01 nM et 5 µM, ou entre 0,1 nM et 500 nM, ou entre 1 nM et 100 nM.

21. Procédé selon la revendication 5, où le contrôle négatif exhibe une constante de dissociation (Kd) pour une liaison au domaine de liaison à un ligand de RAGE supérieure à 5 µM, ou supérieure à 10 µM, ou supérieure à 50 µM, ou supérieure à 100 µM, ou supérieure à 1 mM.

22. Procédé selon la revendication 6, où le contrôle positif exhibe une constante de dissociation (Kd) pour une liaison au domaine de liaison à un ligand de RAGE inférieure à 1 µM, ou inférieure à 200 nM, ou inférieure à 50 nM, ou inférieure à 10 nM.

23. Système de détection de composés qui modulent la liaison d'un ligand à RAGE en utilisant les procédés des revendications 1 à 22, comprenant des récipients, individuellement conditionnés, contenant :
(a) un ligand de RAGE fluorescent, où le ligand fluorescent comprend un polypeptide bêta-amyloïde ;
(b) un polypeptide RAGE comprenant un domaine de liaison à un ligand de RAGE ; et
(c) au moins un réactif d'essai pour la dilution de (a) et (b) en présence d'un composé d'intérêt, de sorte que la liaison du ligand de RAGE fluorescent au polypeptide comprenant le domaine de liaison à un ligand de RAGE puisse être quantifiée.

24. Système selon la revendication 23, comprenant en outre un dispositif pour mesurer la polarisation du ligand fluorescent.

25. Système selon la revendication 23, comprenant en outre un composé comprenant une affinité de liaison prédéterminée pour le domaine de liaison à un ligand de RAGE.

26. Système selon la revendication 25, où le composé comprenant une affinité de liaison prédéterminée pour le domaine de liaison à un ligand de RAGE ne se lie pas essentiellement au domaine de liaison de RAGE, si bien que le second composé fonctionne comme un contrôle négatif pour la mesure de la liaison du domaine de liaison à un ligand de RAGE.

27. Système selon la revendication 25, où le second composé comprend une affinité de liaison substantielle pour le domaine de liaison de RAGE, si bien que le second composé fonctionne comme un contrôle positif pour la mesure de la liaison du domaine de liaison à un ligand de RAGE.

28. Système selon la revendication 23, comprenant en outre un second polypeptide qui est lié à, ou peut former un complexe avec, le polypeptide RAGE.

29. Système selon la revendication 28, où le second polypeptide comprend un anticorps qui reconnaît RAGE ou un fragment de RAGE.


### SEQUENCE LISTING

<110> TransTech Pharma, Inc.
<120> Fluorescence Polarization Assay
<130> 41305-313013
<150> 60/554,183
   <151> 2004-03-18
<160> 7
<170> PatentIn version 3.3
<210> 1
   <211> 404
   <212> PRT
   <213> Homo sapiens
<400> 1
   Gly Ala Ala Gly Thr Ala Val Gly Ala Trp Val Leu Val Leu Ser Leu
   1               5                   10                  15
   Trp Gly Ala Val Val Gly Ala Gln Asn Ile Thr Ala Arg Ile Gly Glu
               20                  25                  30
   Pro Leu Val Leu Lys Cys Lys Gly Ala Pro Lys Lys Pro Pro Gln Arg
           35                  40                  45
   Leu Glu Trp Lys Leu Asn Thr Gly Arg Thr Glu Ala Trp Lys Val Leu
       50                  55                  60
   Ser Pro Gln Gly Gly Gly Pro Trp Asp Ser Val Ala Arg Val Leu Pro
   65                  70                  75                  80
   Asn Gly Ser Leu Phe Leu Pro Ala Val Gly Ile Gln Asp Glu Gly Ile
                   85                  90                  95
   Phe Arg Cys Gln Ala Met Asn Arg Asn Gly Lys Glu Thr Lys Ser Asn
               100                 105                 110
   Tyr Arg Val Arg Val Tyr Gln Ile Pro Gly Lys Pro Glu Ile Val Asp
           115                 120                 125
   Ser Ala Ser Glu Leu Thr Ala Gly Val Pro Asn Lys Val Gly Thr Cys
       130                 135                 140
   Val Ser Glu Gly Ser Tyr Pro Ala Gly Thr Leu Ser Trp His Leu Asp
   145                 150                 155                 160
   Gly Lys Pro Leu Val Pro Asn Glu Lys Gly Val Ser Val Lys Glu Gln
                   165                 170                 175
   Thr Arg Arg His Pro Glu Thr Gly Leu Phe Thr Leu Gln Ser Glu Leu
               180                 185                 190
   Met Val Thr Pro Ala Arg Gly Gly Asp Pro Arg Pro Thr Phe Ser Cys
           195                 200                 205
   Ser Phe Ser Pro Gly Leu Pro Arg His Arg Ala Leu Arg Thr Ala Pro
       210                 215                 220
   Ile Gln Pro Arg Val Trp Glu Pro Val Pro Leu Glu Glu Val Gln Leu
   225                 230                 235                 240
   Val Val Glu Pro Glu Gly Gly Ala Val Ala Pro Gly Gly Thr Val Thr
                   245                 250                 255
   Leu Thr Cys Glu Val Pro Ala Gln Pro Ser Pro Gln Ile His Trp Met
               260                 265                 270
   Lys Asp Gly Val Pro Leu Pro Leu Pro Pro Ser Pro Val Leu Ile Leu
           275                 280                 285
   Pro Glu Ile Gly Pro Gln Asp Gln Gly Thr Tyr Ser Cys Val Ala Thr
       290                 295                 300
   His Ser Ser His Gly Pro Gln Glu Ser Arg Ala Val Ser Ile Ser Ile
   305                 310                 315                 320
   Ile Glu Pro Gly Glu Glu Gly Pro Thr Ala Gly Ser Val Gly Gly Ser
                   325                 330                 335
   Gly Leu Gly Thr Leu Ala Leu Ala Leu Gly Ile Leu Gly Gly Leu Gly
               340                 345                 350
   Thr Ala Ala Leu Leu Ile Gly Val Ile Leu Trp Gln Arg Arg Gln Arg
           355                 360                 365
   Arg Gly Glu Glu Arg Lys Ala Pro Glu Asn Gln Glu Glu Glu Glu Glu
       370                 375                 380
   Arg Ala Glu Leu Asn Gln Ser Glu Glu Pro Glu Ala Gly Glu Ser Ser
   385                 390                 395                 400
   Thr Gly Gly Pro
<210> 2
   <211> 339
   <212> PRT
   <213> Homo sapiens
<400> 2
   Gly Ala Ala Gly Thr Ala Val Gly Ala Trp Val Leu Val Leu Ser Leu
   1               5                   10                  15
   Trp Gly Ala Val Val Gly Ala Gln Asn Ile Thr Ala Arg Ile Gly Glu
               20                  25                  30
   Pro Leu Val Leu Lys Cys Lys Gly Ala Pro Lys Lys Pro Pro Gln Arg
           35                  40                  45
   Leu Glu Trp Lys Leu Asn Thr Gly Arg Thr Glu Ala Trp Lys Val Leu
       50                  55                  60
   Ser Pro Gln Gly Gly Gly Pro Trp Asp Ser Val Ala Arg Val Leu Pro
   65                  70                  75                  80
   Asn Gly Ser Leu Phe Leu Pro Ala Val Gly Ile Gln Asp Glu Gly Ile
                   85                  90                  95
   Phe Arg Cys Gln Ala Met Asn Arg Asn Gly Lys Glu Thr Lys Ser Asn
               100                 105                 110
   Tyr Arg Val Arg Val Tyr Gln Ile Pro Gly Lys Pro Glu Ile Val Asp
           115                 120                 125
   Ser Ala Ser Glu Leu Thr Ala Gly Val Pro Asn Lys Val Gly Thr Cys
       130                 135                 140
   Val Ser Glu Gly Ser Tyr Pro Ala Gly Thr Leu Ser Trp His Leu Asp
   145                 150                 155                 160
   Gly Lys Pro Leu Val Pro Asn Glu Lys Gly Val Ser Val Lys Glu Gln
                   165                 170                 175
   Thr Arg Arg His Pro Glu Thr Gly Leu Phe Thr Leu Gln Ser Glu Leu
               180                 185                 190
   Met Val Thr Pro Ala Arg Gly Gly Asp Pro Arg Pro Thr Phe Ser Cys
           195                 200                 205
   Ser Phe Ser Pro Gly Leu Pro Arg His Arg Ala Leu Arg Thr Ala Pro
       210                 215                 220
   Ile Gln Pro Arg Val Trp Glu Pro Val Pro Leu Glu Glu Val Gln Leu
   225                 230                 235                 240
   Val Val Glu Pro Glu Gly Gly Ala Val Ala Pro Gly Gly Thr Val Thr
                   245                 250                 255
   Leu Thr Cys Glu Val Pro Ala Gln Pro Ser Pro Gln Ile His Trp Met
               260                 265                 270
   Lys Asp Gly Val Pro Leu Pro Leu Pro Pro Ser Pro Val Leu Ile Leu
           275                 280                 285
   Pro Glu Ile Gly Pro Gln Asp Gln Gly Thr Tyr Ser Cys Val Ala Thr
       290                 295                 300
   His Ser Ser His Gly Pro Gln Glu Ser Arg Ala Val Ser Ile Ser Ile
   305                 310                 315                 320
   Ile Glu Pro Gly Glu Glu Gly Pro Thr Ala Gly Ser Val Gly Gly Ser
                   325                 330                 335
   Gly Leu Gly
<210> 3
   <211> 112
   <212> PRT
   <213> Homo sapiens
<400> 3
   Ala Gln Asn Ile Thr Ala Arg Ile Gly Glu Pro Leu Val Leu Lys Cys
   1               5                   10                  15
   Lys Gly Ala Pro Lys Lys Pro Pro Gln Arg Leu Glu Trp Lys Leu Asn
               20                  25                  30
   Thr Gly Arg Thr Glu Ala Trp Lys Val Leu Ser Pro Gln Gly Gly Gly
           35                  40                  45
   Pro Trp Asp Ser Val Ala Arg Val Leu Pro Asn Gly Ser Leu Phe Leu
       50                  55                  60
   Pro Ala Val Gly Ile Gln Asp Glu Gly Ile Phe Arg Cys Gln Ala Met
   65                  70                  75                  80
   Asn Arg Asn Gly Lys Glu Thr Lys Ser Asn Tyr Arg Val Arg Val Tyr
                   85                  90                  95
   Gln Ile Pro Gly Lys Pro Glu Ile Val Asp Ser Ala Ser Glu Leu Thr
               100                 105                 110
<210> 4
   <211> 30
   <212> PRT
   <213> Homo sapiens
<400> 4
   Ala Gln Asn Ile Thr Ala Arg Ile Gly Glu Pro Leu Val Leu Lys Cys
   1               5                   10                  15
   Lys Gly Ala Pro Lys Lys Pro Pro Gln Arg Leu Glu Trp Lys
               20                  25                  30
<210> 5
   <211> 40
   <212> PRT
   <213> Homo sapiens
<400> 5
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val
           35                  40
<210> 6
   <211> 42
   <212> PRT
   <213> Homo sapiens
<400> 6
<210> 7
   <211> 339
   <212> PRT
   <213> Artificial Sequence
<220>
   <223> Modified peptide sequence
<400> 7
   Met Ala Ala Gly Thr Ala Val Gly Ala Trp Val Leu Val Leu Ser Leu
   1               5                   10                  15
   Trp Gly Ala Val Val Gly Ala Gln Asn Ile Thr Ala Arg Ile Gly Glu
               20                  25                  30
   Pro Leu Val Leu Lys Cys Lys Gly Ala Pro Lys Lys Pro Pro Gln Arg
           35                  40                  45
   Leu Glu Trp Lys Leu Asn Thr Gly Arg Thr Glu Ala Trp Lys Val Leu
       50                  55                  60
   Ser Pro Gln Gly Gly Gly Pro Trp Asp Ser Val Ala Arg Val Leu Pro
   65                  70                  75                  80
   Asn Gly Ser Leu Phe Leu Pro Ala Val Gly Ile Gln Asp Glu Gly Ile
                   85                  90                  95
   Phe Arg Cys Gln Ala Met Asn Arg Asn Gly Lys Glu Thr Lys Ser Asn
               100                 105                 110
   Tyr Arg Val Arg Val Tyr Gln Ile Pro Gly Lys Pro Glu Ile Val Asp
           115                 120                 125
   Ser Ala Ser Glu Leu Thr Ala Gly Val Pro Asn Lys Val Gly Thr Cys
       130                 135                 140
   Val Ser Glu Gly Ser Tyr Pro Ala Gly Thr Leu Ser Trp His Leu Asp
   145                 150                 155                 160
   Gly Lys Pro Leu Val Pro Asn Glu Lys Gly Val Ser Val Lys Glu Gln
                   165                 170                 175
   Thr Arg Arg His Pro Glu Thr Gly Leu Phe Thr Leu Gln Ser Glu Leu
               180                 185                 190
   Met Val Thr Pro Ala Arg Gly Gly Asp Pro Arg Pro Thr Phe Ser Cys
           195                 200                 205
   Ser Phe Ser Pro Gly Leu Pro Arg His Arg Ala Leu Arg Thr Ala Pro
       210                 215                 220
   Ile Gln Pro Arg Val Trp Glu Pro Val Pro Leu Glu Glu Val Gln Leu
   225                 230                 235                     240
   Val Val Glu Pro Glu Gly Gly Ala Val Ala Pro Gly Gly Thr Val Thr
                   245                 250                 255
   Leu Thr Cys Glu Val Pro Ala Gln Pro Ser Pro Gln Ile His Trp Met
               260                 265                 270
   Lys Asp Gly Val Pro Leu Pro Leu Pro Pro Ser Pro Val Leu Ile Leu
           275                 280                 285
   Pro Glu Ile Gly Pro Gln Asp Gln Gly Thr Tyr Ser Cys Val Ala Thr
       290                 295                 300
   His Ser Ser His Gly Pro Gln Glu Ser Arg Ala Val Ser Ile Ser Ile
   305                 310                 315                     320
   Ile Glu Pro Gly Glu Glu Gly Pro Thr Ala Gly Ser Val Gly Gly Ser
                   325                 330                 335
   Gly Leu Gly